# EUROPEAN PATENT SPECIFICATION

(11) **EP 2 059 122 B1**
(45) Date of publication and mention of the grant of the patent: **20.06.2012**
(21) Application number: 07841481.0
(22) Date of filing: 28.08.2007
(51) Int. Cl.: C12N 9/98

(54) **METHODS AND COMPOSITIONS RELATED TO APOBEC-1 EXPRESSION**
VERFAHREN UND ZUSAMMENSETZUNGEN IN VERBINDUNG MIT DER EXPRESSION VON APOBEC-1
PROCÉDÉS ET COMPOSITIONS EN RAPPORT AVEC L'EXPRESSION D'APOBEC-I

(30) Priority: 28.08.2006 US 823727 P
(43) Date of publication of application: 20.05.2009
(73) Proprietor: University of Rochester, Rochester, NY 14642 (US)
(72) Inventor: SMITH, Harold, C., Rochester, NY 14623 (US)
(74) Representative: Leissler-Gerstl, Gabriele
(86) International application number: PCT/US2007/077009
(87) International publication number: WO 2008/027899

(56) References cited:
- WO-A-2004/013160
- US-A1- 2004 115 184
- US-A1- 2006 222 657
- SCHWARTZ J ET AL: "PEPTIDE-MEDIATED CELLULAR DELIVERY" CURRENT OPINION IN MOLECULAR THERAPEUTICS, CURRENT DRUGS, LONDON, GB, vol. 2, no. 2, 1 April 2000 (2000-04-01), pages 162-167, XP001037249 ISSN: 1464-8431
- BARKA T. ET AL: 'Production of Cell Lines Secreting TAT Fusion Proteins' J. HISTOCHEM. & CYTOCHEM. vol. 52, no. 4, 2004, pages 469 - 477, XP008104339
- HUGHES S.D. ET AL: 'Gene Transfer of Cytidine Deaminase opoBEC-1 Lowers Lipoprotein(a) in Transgenic Mice and Induces Apolipoprotein B Editing in Rabbits' HUMAN GENE THERAPY vol. 7, no. 1, 2002, pages 39 - 49, XP009050710
- YANG Y. ET AL.: 'Apolipoprotein B mRN-Editing and the Reduction in Synthesis and Secretion of the Aterogenic Risk Factor, Apolipoprotein B 100 can Be Effectively Targeted through TAT-Mediated Protein Transduction' MOL. PHARMACOL. vol. 61, no. 2, 2002, pages 269 - 276, XP003010308
- YAMANAKA S. ET AL.: 'Apolipoprotein B mRNA-editing protein induces hepatocellular carcinoma and dysplasia in transgenic animals' PNAS vol. 92, no. 18, 1995, pages 8483 - 8487, XP002289792
- CHUANG ET AL.: 'Pharmaceutcial Strategies Utilizing Recombinant Human Serum Albumin' PHARM. RES. vol. 19, no. 5, 2002, pages 569 - 577, XP009007551

### SEQUENCE LISTING

<110> university of Rochester SMITH, Harold
<120> compositions and Methods Related to APOBEC-1 Expression
<130> 21108.0077P1
<140> PCT/US07/77009
   <141> 2007-08-28
<150> 60/823,727
   <151> 2006-08-26
<160> 14
<170> FastSEQ for windows version 4.0
<210> 1
   <211> 2215
   <212> DNA
   <213> Artificial Sequence
<220>
   <223> Description of Artificial Sequence; note = synthetic construct
<400> 1
   agcttttctc ttctgtcaac cccacacgcc tttggcacaa tgaagtgggt aacctttatt 60
   tcccttcttt ttctctttag ctcggcttat tccaggggtg tgtttcgtcg agatgcacac 120
   aagagtgagg ttgctcatcg gtttaaagat ttgggagaag aaaatttcaa agccttggtg 180
   ttgattgcct ttgctcagta tcttcagcag tgtccatttg aagatcatgt aaaattagtg 240
   aatgaagtaa ctgaatttgc aaaaacatgt gttgctgatg agtcagctga aaattgtgac 300
   aaatcacttc ataccctttt tggagacaaa ttatgcacag ttgcaactct tcgtgaaacc 360
   tatggtgaaa tggctgactg ctgtgcaaaa caagaacctg agagaaatga atgcttcttg 420
   caacacaaag atgacaaccc aaacctcccc cgattggtga gaccagaggt tgatgtgatg 480
   tgcactgctt ttcatgacaa tgaagagaca tttttgaaaa aatacttata tgaaattgcc 540
   agaagacatc cttactttta tgccccggaa ctccttttct ttgctaaaag gtataaagct 600
   gcttttacag aatgttgcca agctgctgat aaagctgcct gcctgttgcc aaagctcgat 660
   gaacttcggg atgaagggaa ggcttcgtct gccaaacaga gactcaagtg tgccagtctc 720
   caaaaatttg gagaaagagc tttcaaagca tgggcagtag ctcgcctgag ccagagattt 780
   cccaaagctg agtttgcaga agtttccaag ttagtgacag atcttaccaa agtccacacg 840
   gaatgctgcc atggagatct gcttgaatgt gctgatgaca gggcggacct tgccaagtat 900
   atctgtgaaa atcaagattc gatctccagt aaactgaagg aatgctgtga aaaacctctg 960
   ttggaaaaat cccactgcat tgccgaagtg gaaaatgatg agatgcctgc tgacttgcct 1020
   tcattagctg ctgattttgt tgaaagtaag gatgtttgca aaaactatgc tgaggcaaag 1080
   gatgtcttcc tgggcatgtt tttgtatgaa tatgcaagaa ggcatcctga ttactctgtc 1140
   gtgctgctgc tgagacttgc caagacatat gaaaccactc tagagaagtg ctgtgccgct 1200
   gcagatcctc atgaatgcta tgccaaagtg ttcgatgaat ttaaacctct tgtggaagag 1260
   cctcagaatt taatcaaaca aaattgtgag ctttttgagc agcttggaga gtacaaattc 1320
   cagaatgcgc tattagttcg ttacaccaag aaagtacccc aagtgtcaac tccaactctt 1380
   gtagaggtct caagaaacct aggaaaagtg ggcagcaaat gttgtaaaca tcctgaagca 1440
   aaaagaatgc cctgtgcaga agactatcta tccgtggtcc tgaaccagtt atgtgtgttg 1500
   catgagaaaa cgccagtaag tgacagagtc accaaatgct gcacagaatc cttggtgaac 1560
   aggcgaccat gcttttcagc tctggaagtc gatgaaacat acgttcccaa agagtttaat 1620
   gctgaaacat tcaccttcca tgcagatata tgcacacttt ctgagaagga gagacaaatc 1680
   aagaaacaaa ctgcacttgt tgagctcgtg aaacacaagc ccaaggcaac aaaagagcaa 1740
   ctgaaagctg ttatggatga tttcgcagct tttgtagaga agtgctgcaa ggctgacgat 1800
   aaggagacct gctttgccga ggagggtaaa aaacttgttg ctgcaagtca agctgcctta 1860
   ggcttataac atctacattt aaaagcatct cagcctacca tgagaataag agaaagaaaa 1920
   tgaagatcaa aagcttattc atctgttttc tttttcgttg gtgtaaagcc aacaccctgt 1980
   ctaaaaaaca taaatttctt taatcatttt gcctcttttc tctgtgcttc aattaataaa 2040
   aaatggaaag aatctaatag agtggtacag cactgttatt tttcaaagat gtgttgctat 2100
   cctgaaaatt ctgtaggttc tgtggaagtt ccagtgttct ctcttattcc acttcggtag 2160
   aggatttcta gtttctgtgg gctaattaaa taaatcacta atactcttct aagtt      2215
<210> 2
   <211> 39
   <212> DNA
   <213> Artificial sequence
<220>
   <223> Description of Artificial sequence; note = synthetic construct
<400> 2
   agcttttctc ttctgtcaac cccacacgcc tttggcaca 39
<210> 3
   <211> 72
   <212> DNA
   <213> Artificial sequence
<220>
   <223> Description of Artificial sequence; note = synthetic construct
<400> 3
   atgaagtggg taacctttat ttcccttctt tttctcttta gctcggctta ttccaggggt 60
   gtgtttcgtc ga 72
<210> 4
   <211> 1754
   <212> DNA
   <213> Artificial Sequence
<220>
   <223> Description of Artificial Sequence; note = synthetic construct
<400> 4
   gatgcacaca agagtgaggt tgctcatcgg tttaaagatt tgggagaaga aaatttcaaa 60
   gccttggtgt tgattgcctt tgctcagtat cttcagcagt gtccatttga agatcatgta 120
   aaattagtga atgaagtaac tgaatttgca aaaacatgtg ttgctgatga gtcagctgaa 180
   aattgtgaca aatcacttca tacccttttt ggagacaaat tatgcacagt tgcaactctt 240
   cgtgaaacct atggtgaaat ggctgactgc tgtgcaaaac aagaacctga gagaaatgaa 300
   tgcttcttgc aacacaaaga tgacaaccca aacctccccc gattggtgag accagaggtt 360
   gatgtgatgt gcactgcttt tcatgacaat gaagagacat ttttgaaaaa atacttatat 420
   gaaattgcca gaagacatcc ttacttttat gccccggaac tccttttctt tgctaaaagg 480
   tataaagctg cttttacaga atgttgccaa gctgctgata aagctgcctg cctgttgcca 540
   aagctcgatg aacttcggga tgaagggaag gcttcgtctg ccaaacagag actcaagtgt 600
   gccagtctcc aaaaatttgg agaaagagct ttcaaagcat gggcagtagc tcgcctgagc 660
   cagagatttc ccaaagctga gtttgcagaa gtttccaagt tagtgacaga tcttaccaaa 720
   gtccacacgg aatgctgcca tggagatctg cttgaatgtg ctgatgacag ggcggacctt 780
   gccaagtata tctgtgaaaa tcaagattcg atctccagta aactgaagga atgctgtgaa 840
   aaacctctgt tggaaaaatc ccactgcatt gccgaagtgg aaaatgatga gatgcctgct 900
   gacttgcctt cattagctgc tgattttgtt gaaagtaagg atgtttgcaa aaactatgct 960
   gaggcaaagg atgtcttcct gggcatgttt ttgtatgaat atgcaagaag gcatcctgat 1020
   tactctgtcg tgctgctgct gagacttgcc aagacatatg aaaccactct agagaagtgc 1080
   tgtgccgctg cagatcctca tgaatgctat gccaaagtgt tcgatgaatt taaacctctt 1140
   gtggaagagc ctcagaattt aatcaaacaa aattgtgagc tttttgagca gcttggagag 1200
   tacaaattcc agaatgcgct attagttcgt tacaccaaga aagtacccca agtgtcaact 1260
   ccaactcttg tagaggtctc aagaaaccta ggaaaagtgg gcagcaaatg ttgtaaacat 1320
   cctgaagcaa aaagaatgcc ctgtgcagaa gactatctat ccgtggtcct gaaccagtta 1380
   tgtgtgttgc atgagaaaac gccagtaagt gacagagtca ccaaatgctg cacagaatcc 1440
   ttggtgaaca ggcgaccatg cttttcagct ctggaagtcg atgaaacata cgttcccaaa 1500
   gagtttaatg ctgaaacatt caccttccat gcagatatat gcacactttc tgagaaggag 1560
   agacaaatca agaaacaaac tgcacttgtt gagctcgtga aacacaagcc caaggcaaca 1620
   aaagagcaac tgaaagctgt tatggatgat ttcgcagctt ttgtagagaa gtgctgcaag 1680
   gctgacgata aggagacctg ctttgccgag gagggtaaaa aacttgttgc tgcaagtcaa 1740
   gctgccttag gctt 1754
<210> 5
   <211> 350
   <212> DNA
   <213> Artificial sequence
<220>
   <223> Description of Artificial sequence; note = synthetic construct
<400> 5
   ataacatcta catttaaaag catctcagcc taccatgaga ataagagaaa gaaaatgaag 60
   atcaaaagct tattcatctg ttttcttttt cgttggtgta aagccaacac cctgtctaaa 120
   aaacataaat ttctttaatc attttgcctc ttttctctgt gcttcaatta ataaaaaatg 180
   gaaagaatct aatagagtgg tacagcactg ttatttttca aagatgtgtt gctatcctga 240
   aaattctgta ggttctgtgg aagttccagt gttctctctt attccacttc ggtagaggat 300
   ttctagtttc tgtgggctaa ttaaataaat cactaatact cttctaagtt 350
<210> 6
   <211> 609
   <212> PRT
   <213> Artificial Sequence
<220>
   <223> Description of Artificial Sequence; note = synthetic construct
<400> 6
<210> 7
   <211> 236
   <212> PRT
   <213> Artificial sequence
<220>
   <223> Description of Artificial sequence; note = synthetic construct
<400> 7
   Met Thr Ser Glu Lys Gly Pro Ser Thr Gly Asp Pro Thr Leu Arg Arg
    1               5                  10                  15
   Arg Ile Glu Pro Trp Glu Phe Asp Val Phe Tyr Asp Pro Arg Glu Leu
               20                  25                  30
   Arg Lys Glu Ala Cys Leu Leu Tyr Glu Ile Lys Trp Gly Met Ser Arg
           35                  40                  45
   Lys Ile Trp Arg Ser Ser Gly Lys Asn Thr Thr Asn His Val Glu Val
       50                  55                  60
   Asn Phe Ile Lys Lys Phe Thr Ser Glu Arg Asp Phe His Pro Ser Ile
   65                  70                  75                  80
   Ser Cys Ser Ile Thr Trp Phe Leu Ser Trp Ser Pro Cys Trp Glu Cys
                   85                  90                  95
   Ser Gln Ala Ile Arg Glu Phe Leu Ser Arg His Pro Gly Val Thr Leu
               100                 105                 110
   Val Ile Tyr Val Ala Arg Leu Phe Trp His Met Asp Gln Gln Asn Arg
           115                 120                 125
   Gln Gly Leu Arg Asp Leu Val Asn Ser Gly Val Thr Ile Gln Ile Met
       130                 135                 140
   Arg Ala Ser Glu Tyr Tyr His Cys Trp Arg Asn Phe Val Asn Tyr Pro
   145                 150                 155                 160
   Pro Gly Asp Glu Ala His Trp Pro Gln Tyr Pro Pro Leu Trp Met Met
                   165                 170                 175
   Leu Tyr Ala Leu Glu Leu His Cys Ile Ile Leu Ser Leu Pro Pro Cys
               180                 185                 190
   Leu Lys Ile Ser Arg Arg Trp Gln Asn His Leu Thr Phe Phe Arg Leu
           195                 200                 205
   His Leu Gln Asn Cys His Tyr Gln Thr Ile Pro Pro His Ile Leu Leu
       210                 215                 220
   Ala Thr Gly Leu Ile His Pro Ser Val Ala Trp Arg
   225                 230                 235
<210> 8
   <211> 711
   <212> DNA
   <213> Artificial Sequence
<220> -
   <223> Description of Artificial Sequence; note = synthetic construct
<400> 8
   atgacttctg agaaaggtcc ttcaaccggt gaccccactc tgaggagaag aatcgaaccc 60
   tgggagtttg acgtcttcta tgaccccaga gaacttcgta aagaggcctg tctgctctac 120
   gaaatcaagt ggggcatgag ccggaagatc tggcgaagct caggcaaaaa caccaccaat 180
   cacgtggaag ttaattttat aaaaaaattt acgtcagaaa gagattttca cccatccatc 240
   agctgctcca tcacctggtt cttgtcctgg agtccctgct gggaatgctc ccaggctatt 300
   agagagtttc tgagtcggca ccctggtgtg actctagtga tctacgtagc tcggcttttt 360
   tggcacatgg atcaacaaaa tcggcaaggt ctcagggacc ttgttaacag tggagtaact 420
   attcagatta tgagagcatc agagtattat cactgctgga ggaattttgt caactaccca 480
   cctggggatg aagctcactg gccacaatac ccacctctgt ggatgatgtt gtacgcactg 540
   gagctgcact gcataattct aagtcttcca ccctgtttaa agatttcaag aagatggcaa 600
   aatcatctta catttttcag acttcatctt caaaactgcc attaccaaac gattccgcca 660
   cacatccttt tagctacagg gctgatacat ccttctgtgg cttggagatg a 711
<210> 9
   <211> 229
   <212> PRT
   <213> Artificial sequence
<220>
   <223> Description of Artificial Sequence; note = synthetic construct
<400> 9
<210> 10
   <211> 689
   <212> DNA
   <213> Artificial Sequence
<220>
   <223> Description of Artificial Sequence; note = synthetic construct
<400> 10
   atgagttccg asacaggccc tgtagctgtt gatcccactc tgaggagaag aattgagccc 60
   cacgagtttg aagtcttctt tgacccccgg gaacttcgga aagagacctg tctgctgtat 120
   gagatcaact gggaggaagg cacagcatct ggcgacacac gagccaaaac accaacaaac 180
   acgttgaagt caatttcata gaaaaattta ctacagaaag atacttttgt ccaaacacca 240
   gatgctccat tacctggttc ctgtcctgga gtccctgtgg ggagtgctcc agggccatta 300
   cagaattttt gagccgatac ccccatgtaa ctctgtttat ttatatagca cggctttatc 360
   accacgcaga tcctcgaaat cggcaaggac tcagggacct tattagcagc ggtgttacta 420
   tccagatcat gacggagcaa gagtctggct actgctggag gaattttgtc aactactccc 480
   cttcgaatga agctcattgg ccaaggtacc cccatctgtg ggtgaggctg tacgtactgg 540
   aactctactg catcatttta ggacttccac cctgtttaaa tattttaaga agaaaacaac 600
   ctcaactcac gtttttcacg attgctcttc aaagctgcca ttaccaaagg ctaccacccc 660
   acatcctgtg ggccacaggg ttgaaatga                                   689
<210> 11
   <211> 229
   <212> PRT
   <213> Artificial Sequence
<220>
   <223> Description of Artificial Sequence; note = synthetic construct
<400> 11
   Met Ser Ser Glu Thr Gly Pro Val Ala Val Asp Pro Thr Leu Arg Arg
   1               5                   10                  15
   Arg Ile Glu Pro His Glu Phe Glu Val Phe Phe Asp Pro Arg Glu Leu
               20                  25                  30
   Arg Lys Glu Thr Cys Leu Leu Tyr Glu Ile Asn Trp Gly Gly Arg His
           35                  40                  45
   Ser Val Trp Arg His Thr Ser Gln Asn Thr Ser Asn His Val Glu Val
       50                  55                  60
   Asn Phe Leu Glu Lys Phe Thr Thr Glu Arg Tyr Phe Arg Pro Asn Thr
   65                  70                  75                  80
   Arg Cys Ser Ile Thr Trp Phe Leu Ser Trp Ser Pro Cys Gly Glu Cys
                   85                  90                  95
   Ser Arg Ala Ile Thr Glu Phe Leu Ser Arg His Pro Tyr Val Thr Leu
               100                 105                 110
   Phe Ile Tyr Ile Ala Arg Leu Tyr His His Thr Asp Gln Arg Asn Arg
           115                 120                 125
   Gln Gly Leu Arg Asp Leu Ile Ser Ser Gly Val Thr Ile Gln Ile Met
       130                 135                 140
   Thr Glu Gln Glu Tyr Cys Tyr Cys Trp Arg Asn Phe Val Asn Tyr Pro
   145                 150                 155                 160
   Pro Ser Asn Glu Ala Tyr Trp Pro Arg Tyr Pro His Leu Trp Val Lys
                   165                 170                 175
   Leu Tyr Val Leu Glu Leu Tyr Cys Ile Ile Leu Gly Leu Pro Pro Cys
               180                 185                 190
   Leu Lys Ile Leu Arg Arg Lys Gln Pro Gln Leu Thr Phe Phe Thr Ile
           195                 200                 205
   Thr Leu Gln Thr Cys His Tyr Gln Arg Ile Pro Pro His Leu Leu Trp
       210                 215                 220
   Ala Thr Gly Leu Lys
   225
<210> 12
   <211> 689
   <212> DNA
   <213> Artificial sequence
<220>
   <223> Description of Artificial Sequence; note = synthetic construct
<400> 12
   atgagttccg agacaggccc tgtagctgtt gatcccactc tgaggagaag aattgagccc 60
   cacgagtttg aagtcttctt tgacccccgg gagcttcgga aagagacctg tctgctgtat 120
   gagatcaact ggggtggaag gcacagtgtc tggcgacaca cgagccaaaa caccagcaac 180
   cacgttgaag tcaacttctt agaaaaattt actacagaaa gatactttcg tccgaacacc 240
   agatgctcca ttacctggtt cctgtcctgg agtccctgcg gggagtgctc cagggccatt 300
   acagagtttc tgagccgaca cccctatgta ctctgtttat ttacatagca cggctttatc 360
   accacacgga tcagcgaaac cgccaaggac tcagggacct tattagcagc ggtgtgacta 420
   tccagatcat gacagagcaa gagtattgtt actgctggag gaatttcgtc aactaccccc 480
   cttcaaacga agcttattgg ccaaggtacc cccatctgtg ggtgaaactg tatgtattgg 540
   agctctactg catcatttta ggacttccac cctgtttaaa aattttaaga agaaagcaac 600
   ctcaactcac gtttttcaca attactcttc aaacctgcca ttaccaaagg ataccacccc 660
   atctcctttg ggctacaggg ttgaaatga                                   689
<210> 13
   <211> 9
   <212> PRT
   <213> Artificial sequence
<220>
   <223> Description of Artificial Sequence; note = synthetic construct
<400> 13
   Arg Lys Lys Arg Arg Gln Arg Arg Arg
   1 5
<210> 14
   <211> 27
   <212> DNA
   <213> Artificial sequence
<220>
   <223> Description of Artificial sequence; note = synthetic construct
<400> 14
   agaaaaaaaa gaagacaaag aagaaga 27

## Description

### BACKGROUND OF THE INVENTION

Dietary cholesterol is carried into blood from the intestine by a specific carrier protein called apolipoprotein B48 and from liver into the circulation for distribution to other tissues as apolipoprotein B 100 or apoB100 and apolipoprotein B48 depending on the species. Apolipoprotein B is an integral and non-exchangeable structural component of lipoprotein particles referred to as chylomicrons, very low density lipoprotein ("VLDL"), and low density lipoprotein ("LDL"). Apolipoprotein B circulates in human plasma as two isoforms, apolipoprotein B100 and apolipoprotein B48. Apolipoprotein B48 is generated by an RNA editing mechanism which changes codon 2153 (CAA) to a translation stop codon (UAA) (Chen et al., Science 238:363-366 (1987); Powell et al., Cell 50:831-840 (1987)). Editing is a site-specific deamination event catalyzed by apolipoprotein B mRNA editing catalytic subunit 1 (known as APOBEC-1) (Teng et al., Science 260:18116-1819 (1993)) with the help of auxiliary factors (Teng et al., Science 260:18116-1819 (1993); Yang et al., J: Biol.Chem. 272:27700-27706 (1997); Yang et al., Proc. Natl. Acad. Sci. USA 94:13075-13080 (1997); Lellek et al., J. Biol. Chem. 275:19848-19856 (2000); Mehta et al., Mol. Cell. Biol. 20:1846-1854 (2000); Yang et al., J. Biol. Chem. 275:22663-22669 (2000); Blanc et al., J. Biol. Chem. 276:10272-10283 (2001)) as a holoenzyme or editosome (Smith et al. Proc. Natl. Acad. Sci. USA 88:1489-1493 (1991); Harris et al., J. Biol. Chem. 268:7382-7392 (1993)). Apolipoprotein B100 and apolipoprotein B48 play different roles in lipid metabolism, most importantly, apolipoprotein B100-associated lipoproteins (VLDL and LDL) are much more atherogenic than apolipoprotein B48-associated lipoproteins (chylomicrons and their remnants and VLDL).

Specifically, the apolipoprotein B48-associated lipoproteins are cleared from serum more rapidly than the apolipoprotein B100-associated lipoproteins. As a result, apolipoprotein B48-VLDL usually are not present in serum for an amount of time sufficient for serum lipases to convert the VLDL to LDL. In contrast, the apolipoprotein B 100-VLDL are present in the serum for sufficient amounts of time, allowing serum lipases to convert the VLDL to LDL. Elevated serum levels of LDL are of particular biomedical significance as they are associated with an increased risk of atherogenic diseases or disorders. Lipoprotein analyses have shown that the ability of mammalian liver to edit results in a lowering of the VLDL+LDL:HDL ratio. Therefore, it would be desirable to identify an approach for modifying apolipoprotein B editing which would favor an increase in the relative concentration of apolipoprotein B48 in proportion to apolipoprotein B100 (or total apolipoprotein concentration), thereby clearing a greater concentration of lipoproteins from serum and minimizing the atherogenic risks associated with high serum levels of VLDL and LDL.

Current lipid-lowering therapies include statins and bile-acid-binding resins. Statins are competitive inhibitors of hydroxymethylglutaryl-coenzyme A (HMG-CoA) reductase, which catalyzes the committed step in the synthesis of cholesterol (Davignon et al., Can. J. Cardiol. 8:843-64 (1992)). Bile-acid-binding resins sequester bile acids in the intestine, thereby interrupting the enterohepatic circulation of bile acids and increasing the elimination of cholesterol from the body. These are effective therapies for some patients with hyperlipidemia; however, adverse effects have been observed in up to 30% of the patients, suggesting the need for alternative therapies. Mutations in the gene encoding the LDL-receptor or apolipoprotein B can cause a human genetic disease known as familial hypercholesterolemia, characterized by an elevated level of cholesterol and early atherosclerosis due to the defect in LDL-receptor mediated cholesterol uptake by cells (Goldstein et al., In The Metabolic and Molecular Bases of Inherited Disease, Vol. 2., p1981-2030, Scriver et al. (eds.), McGraw-Hill, New York (1995)). Therapy for children with this disorder is needed in order to prevent morbidity or mortality, however the National Cholesterol Education Program (NCEP) recommends consideration of drug treatment only for children 10 years of age or older due to the risk that prolonged drug therapy may impair growth and pubertal development. Developing alternative approaches for lowering serum LDL levels is therefore essential for the sectors of the population still at risk.

Stimulating hepatic apolipoprotein B mRNA editing is a means of reducing serum LDL through the reduction in synthesis and secretion of apolipoprotein B100 containing VLDL. In most mammals (including humans), apolipoprotein B MRNA editing is carried out only in the small intestine. The presence of substantial editing in liver (found in 4 species) is associated with a less atherogenic lipoprotein profile compared with animals that do not have liver editing activity (Greeve et al., J. Lipid Res. 34:1367-1383 (1993)). APOBEC-1 is expressed in all tissues that carry out apolipoprotein B mRNA editing (Teng et al., Science 260:18116-1819 (1993)). Human liver does not express APOBEC-1 but it does express sufficient auxiliary proteins to complement exogenous APOBEC-1 in apolipoprotein B MRNA editing in transfected cells (Teng et al., Science 260:18116-1819 (1993); Sowden et al., Nucleic Acids Res. 26:1644-1652 (1998)).

Transgenic experiments aiming to enhance hepatic editing through apobec-1 gene transfer have shown a marked lowering of plasma apolipoprotein B100 and significant reduction of serum LDL (Teng et al., J. Biol. Chem. 269:29395-29404 (1994); Hughs et al., Hum. Gene Ther. 7:39-49 (1996); Nakamuta et al., J. Biol. Chem. 271:25981-25988 (1996); Kozarsky et al., Hum. Gene Ther. 7:943-957 (1996); Farese et al., Proc. Natl. Acad. Sci. USA 93:6393-6398 (1996); Qian et al., ., Arteriosc. Thromb. Vasc. Biol. 18:1013-1020 (1998); Wu et al., J. Surg. Res. 85:148-157 (1999)). Apolipoprotein B100 is not essential for life as mice that synthesize exclusively apolipoprotein B48 (apolipoprotein B48-only mice) generated through targeted mutagenesis developed normally, were healthy and fertile. Compared with wild-type mice fed on a chow diet, the level of LDL-cholesterol was lower in apolipoprotein B48-only mice (Farese et al., Proc. Natl. Acad. Sci. USA 93:6393-6398 6398 (1996)). However, the induction of apelipoprotein B mRNA editing activity through apobec-1 gene transfer and tissue-specific overexpression poses a significant challenge in that it has induced hepatocellular dysplasia and carcinoma in transgenic mice and rabbits (Yamanaka et al., Proc. Natl. Acad. Sci. USA 92: 8483-8487 (1995); Yamanaka et al., J. Biol. Chem. 271:11506-11510 (1996); Yamanaka et al., Genes & Dev. 11:321-333 (1997)). This was proposed to be due to persistent high levels of APOBEC-1 expression resulting in unregulated and nonspecific mRNA editing (Sowden et al., J. Biol. Chem 271:3011-3017 (1996); Yamanaka et al., Genes & Dev. 11:321-333 (1997); Sowden et al., Nucleic Acids Res. 26:1644-1652 (1998)). Adverse effects were not observed in transgenic animals with low to moderate levels of APOBEC-1 expression (Teng et al., J. Biol. Chem. 269:29395-29404 (1994); Qiarr et al., Arteriosc. Thromb. Vasc. Biol. 18:1013-1020 (1998); Wu et al., J. Surg. Res. 85:148-i57 (1999)).

For these reasons, it would be desirable to identify an approach to achieve apolipoprotein B mRNA editing, where APOBEC-1 acts in the proper location, and does not need to be systemically administered. Moreover, it would be desirable to identify an approach to achieve apolipoprotein B mRNA editing which does not require the production or administration of proteins. The present invention is directed to overcoming these and other deficiencies in the art.

### SUMMARY OF THE INVENTION

In accordance with the purposes of this invention, as embodied and broadly described herein, this invention, in one aspect, relates to methods of expressing APOBEC-1 in a cellas defined in the claimsFor example, disclosed are methods of expressing APOBEC-1 in a cell, comprising bringing into contact a cell and a vector comprising a nucleic acid, wherein the nucleic acid encodes a polypeptide comprising an APOBEC-1 sequence, a secretion sequence and a transduction sequence; whereby the nucleic acid produces APOBEC-1, thereby expressing APOBEC-1 in the cell, and wherein the APOBEC-1 can then be secreted from the cell and transduce a second cell, thereby delivering the polypeptide to the second cell. In some forms of the disclosed methods, a chimeric protein composed of a secretion signal-containing TAT-APOBEC-1 can be expressed in a cell for the purpose of producing and secreting TAT-APOBEC into the circulation. For example, disclosed are methods of expressing signal sequence-TAT-APOBEC-1 in a cell, comprising bringing into contact a cell and a vector comprising a nucleic acid, wherein the nucleic acid encodes a polypeptide comprising a signal sequence-TAT-APOBEC-1 sequence, wherein the signal sequence directs the nascent TAT-APOBEC-1 for translation in association with the endoplasmic reticulum and secretion from the cell.

Also disclosed herein are APOBEC-1 Secretion-Transduction Sequence (STS) polypeptides, nucleic acids, and compositions thereof.

Additional advantages of the invention will be set forth in part in the description which follows, and in part will be obvious from the description, or may be learned by practice of the invention. The advantages of the invention will be realized and attained by means of the elements and combinations particularly pointed out in the appended claims. It is to be understood that both the foregoing general description and the following detailed description are exemplary and explanatory only and are not restrictive of the invention, as claimed.

### BRIEF DESCRIPTION OF THE DRAWINGS

The accompanying drawings, which are incorporated in and constitute a part of this specification, illustrate several embodiments of the invention and together with the description, serve to explain the principles of the invention.

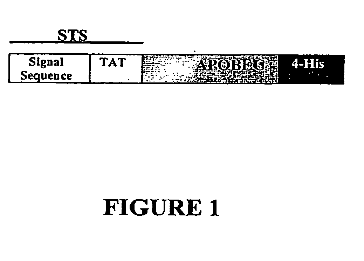

Figure 1 shows an APOBEC-1 Secretion-Transduction Sequence (STS). Shown here is a signal sequence fused to a TAT sequence. The STS polypeptide comprises APOBEC-1 molecule, and is followed by histidine residues.

### DETAILED DESCRIPTION

The APOBEC-1 Secretion Transduction Sequence (STS) polypeptides and nucleic acids described herein are useful in treating various diseases and disorders associated with apolipoprotein B100.

The present invention may be understood more readily by reference to the following detailed description of preferred embodiments of the invention and the Examples included therein and to the Figures and their previous and following description.

Before the present compounds, compositions, articles, devices, and/or methods are disclosed and described, it is to be understood that this invention is not limited to specific synthetic methods, specific recombinant biotechnology methods unless otherwise specified, or to particular reagents unless otherwise specified, as such may, of course, vary. It is also to be understood that the terminology used herein is for the purpose of describing particular embodiments only and is not intended to be limiting.

### DEFINITIONS

As used in the specification and the appended claims, the singular forms "a," "an" and "the" include plural referents unless the context clearly dictates otherwise. Thus, for example, reference to "a pharmaceutical carrier" includes mixtures of two or more such carriers, and the like.

Ranges may be expressed herein as from "about" one particular value, and/or to "about" another particular value. When such a range is expressed, another embodiment includes from the one particular value and/or to the other particular value. Similarly, when values are expressed as approximations, by use of the antecedent "about," it will be understood that the particular value forms another embodiment. It will be further understood that the endpoints of each of the ranges are significant both in relation to the other endpoint, and independently of the other endpoint. It is also understood that there are a number of values disclosed herein, and that each value is also herein disclosed as "about" that particular value in addition to the value itself. For example, if the value "10" is disclosed, then "about 10" is also disclosed. It is also understood that when a value is disclosed that "less than or equal to" the value, "greater than or equal to the value" and possible ranges between values are also disclosed, as appropriately understood by the skilled artisan. For example, if the value "10" is disclosed the "less than or equal to 10"as well as "greater than or equal to 10" is also disclosed.

"Optional" or "optionally" means that the subsequently described event or circumstance may or may not occur, and that the description includes instances where said event or circumstance occurs and instances where it does not.

The terms "higher," "increases," "elevates," "enhances," or "elevation" refer to increases as compared to a control. The terms "low," "lower," "reduces," "suppresses" or "redaction" refers to decreases as compared to a control level. Control levels can be normal *in vivo* levels prior to, or in the absence of, treatment. Thus, the control can be from the same subject prior to treatment or can be an untreated control subject or group thereof.

By "subject" is meant an individual. Preferably, the subject is a mammal such as a primate, and, more preferably, a human. The term "subject" can include domesticated animals, such as cats, dogs, etc., livestock (e.g., cattle, horses, pigs, sheep, goats, etc.), and laboratory animals (e.g., mouse, rabbit, rat, guinea pig, etc.).

The terms "control levels" or "control cells" are defined as the standard by which a change is measured, for example, the controls are not subjected to the experiment, but are instead subjected to a defined set of parameters, or the controls are based on pre- or post-treatment levels.

By "contacting" is meant an instance of exposure of at least one substance to another substance. For example, contacting can include contacting a substance, such as a cell, or cell to a test compound described herein. A cell can be contacted with the test compound, for example, by adding the protein or small molecule to the culture medium (by continuous infusion, by bolus delivery, or by changing the medium to a medium that contains the agent) or by adding the agent to the extracellular fluid *in vivo* (by local delivery, systemic delivery, intravenous injection, bolus delivery, or continuous infusion). The duration of contact with a cell or group of cells is determined by the time the test compound is present at physiologically effective levels or at presumed physiologically effective levels in the medium or extracellular fluid bathing the cell.

By "treatment" and "treating" is meant the medical management of a subject with the intent to cure, ameliorate, stabilize, or prevent a disease, pathological condition, or disorder. This term includes active treatment, that is, treatment directed specifically toward the improvement of a disease, pathological condition, or disorder, and also includes causal treatment, that is, treatment directed toward removal of the cause of the associated disease, pathological condition, or disorder. In addition, this term includes palliative treatment, that is, treatment designed for the relief of symptoms rather than the curing of the disease, pathological condition, or disorder; preventative treatment, that is, treatment directed to minimizing or partially or completely inhibiting the development of the associated disease, pathological condition, or disorder; and supportive treatment, that is, treatment employed to supplement another specific therapy directed toward the improvement of the associated disease, pathological condition, or disorder. It is understood that treatment, while intended to cure, ameliorate, stabilize, or prevent a disease, pathological condition, or disorder, need not actually result in the cure, ameliorization, stabilization or prevention. The effects of treatment can be measured or assessed as described herein and as known in the art as is suitable for the disease, pathological condition, or disorder involved. Such measurements and assessments can be made in qualitative and/or quantitiative terms. Thus, for example, characteristics or features of a disease, pathological condition, or disorder and/or symptoms of a disease, pathological condition, or disorder can be reduced to any effect or to any amount.

By "effective amount" is meant a therapeutic amount needed to achieve the desired result or results, e.g., reducing atherogenic risk, blunting physiological functions, altering the qualitative or quantitative nature of the proteins expressed by cell or tissues, and eliminating or reducing disease causing molecules and/or the mRNA or DNA that encodes them, such as ApoB100.

Herein, "inhibition" or "suppression" means to reduce activity as compared to a control (e.g., activity in the absence of such inhibition). It is understood that inhibition or suppression can mean a slight reduction in activity to the complete ablation of all activity. An "inhibitor" or "suppressor" can be anything that reduces the targeted activity.

Many methods disclosed herein refer to "systems." It is understood that systems can be, for example, cells, columns, or batch processing containers (e.g., culture plates). A system is a set of components, any set of cornponents-that allows for the steps of the method to performed. Typically a system will comprise one or more components, such as a protein(s) or reagent(s).

### GENERAL

Reduction of atherogenic LDL-cholesterol is one of the most well-established measures for management of patients at risk for coronary artery disease (CAD). Many factors contribute to atherogenic disease. However, studies evaluating patients with atherosclerosis concluded that elevated LDL (hypercholesterolemia) was the primary risk factor for disease in 25% of the population. The family of hydrox-ymethyl CoA reductase inhibitors, collectively known as statins, has proven efficacy in the reduction of circulating LDL-cholesterol and reduction in coronary events. The statins are well-tolerated by the majority of patients, but there are significant populations for whom statin therapy has little or no benefit. Among these groups are those with familial hyperlipidemias, severe side effects, typically myopathies, and low tolerance for the statins (therapeutic dose is greater than tolerated dose). The population of patients who derive little or no benefit from statin therapy may be as many a 10% of those with hyperlipidemia (Gibbons et al. (2004) Circulation 109:IV47-58.) This percentage is likely to increase as patients with poor statin tolerance are uncovered during treatment of fatty liver disease and nonalcoholic steatohepatitis (NASH) brought on by obesity and type II diabetes. Treatment of fatty liver disease is essential to avoid cirrhosis of the liver, however, reduction of liver fat requires increased clearance through the VLDL assembly and secretion pathway, and this will increase serum VLDL and inevitably elevate serum LDL-cholesterol.

RNA-editing by APOBEC-1 modulates the abundance of atherogenic apoB100-containing LDL and rapidly cleared apoB48 VLDL. Apolipoprotein B is a non-exchangeable structural component of intestinally-derived chylomicrons and of liver-derived VLDL particles ApoB is translated from a 14 kb mRNA that is transcribed from a single copy gene located on human chromosome 2 (Scott, (1989) Mol Biol Med 6:65-80). A large (apoB100) and small (apoB48) isoform of apoB lipoprotein are expressed by post-transcriptional RNA editing (Powell et al. (1987) Cell 50:831-840, Chen et al. (1987) Science 238:363-366). ApoB mRNA editing involves a site-specific hydrolytic deamination of cytidine at nucleotide 6666 (C6666) to form uridine (Johnson et al. (1993) Biochem Biophys Res Commun 195:1204-1210), thereby creating an in-frame translation stop codon, UAA, from a glutamine codon, CAA (Powell (1987), Chen (1987). The mammalian small intestine constitutively edits ≥85% of apoB mRNA and synthesizes and stores apoB48 for the assembly and secretion of chylomicrons containing dietary lipids (Powell (1987), Chen (1987), Greeve et al. (1993) J Lipid Res 34:1367-1383, Backus et al. (1990) Biochem Biophys Res Commun 170:513-518).

In some mammals (Greeve et al. (1993)), with the notable-exception of humans, apoB mRNA editing also occurs in the liver. Mouse and rat livers regulate apoB mRNA editing thereby modulating the proportion of edited apoB mRNA and the proportion of apoB48 versus apoB100 secreted as VLDL (Greeve et al. (1993), Sparks et al. (1981) Can J Biochem 59:693-699). Hepatic VLDLs are assembled co-translationally with apoB48 or apoB100 and are secreted into the blood from where they are cleared by peripheral tissues, particularly fat and muscle, and the liver. ApoB100 VLDL are converted by serum lipases to low density lipoprotein particles (LDL), an atherogenic risk factor (Corsetti et al. (2003)), whereas apoB48 VLDL lack the LDL receptor binding domain and are cleared more rapidly via chylomicron remnant or apoE surface receptors. The difference in VLDL-half-life in serum (apoB48 VLDL around 10 minutes; apoB100 VLDL around 10 hours) results in apoB100 VLDL metabolism to LDL. When apoB mRNA editing is induced in the human liver, LDL to HDL ratios fall and there is a corresponding reduction in coronary artery disease.

Lipoprotein analyses in twelve mammalian species revealed a high correlation between apoB mRNA-editing in liver and reduced apoB100 VLDL+LDL to HDL ratio in the serum (Greeve (1993)). APOBEC-1 -/- knockout mice expressing a human apoB transgene expressed only apoB100 in their intestine and liver and had sharply elevated serum LDL levels (Hirano, K. et al. (1996) J Biol Chem 271:9887-9890, Nakamuta, M. et al. (1996) J Biol Chem 271:25981-25988).

In addition, low triglyceride secretion rates and larger lipoprotein size were also noted in these animals (Xie et al. (2003) Am J Physiol Gastrointest Liver Physiol 285:G735-746). An APOBEC-1 -/- knockout mice has been obtained, and elevated hepatic triglyceride content in these animals when fed normal chow has been noted, showing that they cannot mobilize lipids from the liver efficiently. These observations show that reduced editing in a species that normally produces hepatic apoB48 can contribute to fatty liver disease as well as atherosclerosis.

Normal ratios of apoB48:apoB100 in serum VLDL and the proportion of serum VLDL and LDL lipoproteins was restored by apobec-1 gene transfer without adverse side effects (Nakamuta, M. et al. (1996), Qian, X. et al. (1998) Arterioscler Thromb Vasc Biol 18,1013-1020, Teng et al. (1994) J Biol Chem 269:29395-29404, Hughes et al. (1996) Hum Gene Ther 7:39-49). From these findings it was realized that APOBEC-1 is the only enzyme capable of editing apoB mRNA. Taken together, the observations that 1) all other auxiliary proteins required for APOBEC-1 site-specific editing are expressed in human liver (Navaratnam et al. (1993) Proc Natl Acad Sci USA 90:222-226, Dance et al. (2002) J Biol Chem 277:12703-12709); 2) editing can be induced in human heptoma HepG2 cells by overexpression of APOBEC-1 (Giannoni, F. et al. (1994) J Biol Chem 269, 5932-5936, Sowden, et al. (1996) J Biol Chem 271, 3011-3017, Teng et al. (1993) Science 260, 1816-1819); and 3) apobec-1 knockout mice acquire normal lipid-profiles by "rescue" with apobec-1 gene therapy led to the discovery that apobec-1 gene therapy can be used to treat atherogenic disease.

The RNA-editing enzyme APOBEC-1 is highly conserved among mammals and requires independently regulated cofactors. The amino acid sequence of APOBEC-1 from several organisms is highly conserved (Teng, B. et al. (1994), Yamanaka, et al. (1994) J Biol Chem 269, 21725-21734, Nakamuta et al. (1995) J Biol Chem 270, 13042-13056, Lau et al. (1994) Proc Natl Acad Sci U S A 91, 8522-8526, Hadjiagapiou, et al. (1994) Nucleic Acids Res 22, 1874-1879). It is a zinc-dependent enzyme whose catalytic domain shares similarity with a variety of nucleoside/nucleotide deaminases (Navaratnam et al. (1993), Anant et al. (1998) Biol Chem 379, 1075-1081, Mian et al. (1998) J Comput Biol 5, 57-72, Wedekind et al. (2003) Trends Genet 19, 207-216). APOBEC-1 is expressed in several tissues, albeit at very low levels compared to liver and intestine, the only two tissues that support apoB mRNA editing (Greeve et al. (1993), Qian et al. (1998), Nakamuta et al. (1995).

TAT-APOBEC can be produced in bacteria, used to transduce rat primary hepatocytes in culture and within 6 hours distributes to the cell nucleus and induces apoB mRNA editing (Yang et al. (2002) Mol Pharmacol 61, 269-276). The increase in editing activity was dose-dependent and was sustained for 72 h following a single dose. Importantly, increasing editing activity in hepatocytes markedly increased the proportion of secreted VLDL assembled on apoB48 while reducing the amount of apoB100. There was no evidence of promiscuous editing activity or changes in cell morphology and, following 72 h after a single dose, the transduced editing activity was completely reversed presumably by normal protein turnover.

Approximately 20% of proteins synthesized in CHO cells are targeted to the endoplasmic reticulum (ER) through a signal peptide (SP) and are secreted. In addition to N-terminal SP, the 3'-untranslated region (3'-UTR) of mRNAs facilitate their targeting to the ER for translation and secretion of the translation product (Partridge et al. Cytotechnology 30, 37-47). Adding these sequences to cDNA results in synthesis and secretion of the protein of interest.

For example, well-characterized secretory signals linked to TAT-HA-APOBEC and gene delivery via AAV2 can be used to generate somatic mosaic expression and secretion of TAT-HA-APOBEC for extracellular distribution and uptake by adjacent hepatocytes in the liver. This is useful because, for example: 1) targeting TAT-HA-APOBEC for secretion can prevent the build up of TAT-HA-APOBEC in the producer cell, thereby averting effects to due to overexpression and promiscuous editing known to arise when APOBEC-1 is overexpressed (Sowden 1998; Yamanaka 1997); 2) secreted TAT-HA-APOBEC can be rapidly diluted in the intrahepatic but extracellular fluids in the Space of Disse, thereby reducing the probability of transduction of the producer cell; 3) hepatocytes can be transduced by TAT-HA-APOBEC and secrete apoB48-containing VLDL in a dose-dependent manner defined by the concentration gradients established around the producer hepatocytes; and 4) the effect of TAT-HA-APOBEC transduction and induction of apoB mRNA editing activity is localized primarily to the liver due to the anatomy of the intrahepatic circulation, the absence of blood cells in the Space ofDisse, the absence of heparin polysaccharides on Kupffer cells and the limited expression of apoB mRNA in other tissues.

Useful effects of the disclosed compositions and methods can be, for example: 1) low level infection with rAA V2-ensuresHsomaticmosaicism that has been shown previously to be safe in animals is infection with this virus does not elicite a significant immune response; 2) secretion of TAT-HA-APOBEC reduces the chances of pathology due to over-expression and editing activity in the hepatocyte producing TAT-HA-APOBEC, 3) transduction of TAT-HA-APOBEC is limited to zones within the liver surrounding the producer cells in which hepatocytes edit some but not all of their apoB mRNA and therefore the liver assembles and secretes some but not all of the VLDL with apoB48. The latter point is an important therapeutic parameter to achieve through titration of viral infection.

Advantages of the disclosed compositions and method can include, for example: 1) it has the highest potential of any therapeutic approach of targeting apoB mRNA-editing without risk of off target effects or tissue pathology; 2) circumvents the expense and process necessary for the production of a TAT-APOBEC protein therapeutic; 3) can be titrated and individualized relative to the desired serum lipid profile and thereafter requires minimal intervention; 4) minimizes drug-drug interaction effects because as a protein, it is metabolized by normal protein turnover; and 5) can reduce serum LDL levels alone and can also be used in combination with statins and other lipid-lowering drugs, enabling the use of lower therapeutic doses and reducing potential side-effects.

### COMPOSITIONS

### STS Polypeptides and Nucleic Acids

Disclosed herein are APOBEC-1 Secretion-Transduction Sequence (STS) polypeptides. "STS polypeptide" refers to a polypeptide comprising an APOBEC-1 sequence, a transduction sequence, and a secretion sequence. "STS nucleic acid" refers to a nucleic acid encoding a polypeptide comprising an APOBEC-1 sequence, a transduction sequence, and a secretion sequence. The STS polypeptides and nucleic acids can be part of a composition as well, herein referred to as "STS compositions."

In some embodiments, the APOBEC-1. sequence, secretion sequence, and transduction sequence can be operably linked. For example, the APOBEC-1 sequence, secretion sequence, and transduction sequence can be contiguous. SEQ ID NO: 1 is an example of an STS polypeptide wherein APOBEC-1, the secretion sequence, and the transduction sequence are contiguous.

A nucleic acid sequence for TAT-HA-APOBEC-1, including the 5' UTR leader, the signal sequence, and the 3' UTR sequence is disclosed in SEQ ID NO:1:

The sequence for the leader (5' UTR) in SEQ ID NO:1 is agcttttctc ttctgtcaac cccacacgcc tttggcaca (SEQ ID NO:2).

The signal sequence in SEQ ID NO:1 is atgaagtgggt aacctttatt tcccttcttt ttctctttag ctcggcttat tccaggggtg tgtttcgtcg a (SEQ ID NO:3).

A mature TAT-HA-APOBEC-1 sequence is SEQ ID NO:4:

A sequence for the 3' UTR of albumin is SEQ ID NO:5:

The albumin 5' UTR plus signal peptide is nucleotides 1-111 of SEQ ID NO: 5. This is followed by TAT-HA-APOBEC and then the 3' UTR of albumin is 1867-2215. Polyadenylation occur as 2215 but there is another site at 2055 and 2078. All of the 5' UTR (111 nt; -33 amino acids) and all of the 3' UTR (349 nt no addition to protein length) are used in the sequence.

An amino acid sequence for TAT-HA-APOBEC-1 is SEQ ID NO:6:

### Apobec-1

Disclosed herein are APOBEC-1 polypeptides and fragments thereof. The full length human APOBEC-1 has an amino acid sequence according to SEQ ID NO: 7 as follows:

This human APOBEC-1 sequence is reported at Genbank Accession No. NP _13001635,

The full length human APOHEC-1 includes a putative bipartite nuclear localization signal between amino acid residues 15-34, a catalytic center between amino acid residues 61-98, and a putative cytoplasmic retention signal between amino acid residues 173-229. A cDNA sequence which encodes the full length human APOBEC- is set forth as SEQ ID NO: 8 as follows:

The full length rat APOBEC-1 has an amino acid sequence according to SEQ ID NO:9 as follows:

This rat APOBEC-1 sequence is reported at Genbank Accession No. P38483, Recombinant studies using rat APOBEC-1 have demonstrated that an N-terminal region, containing the putative nuclear localization signal, is required for nuclear distribution of APOBEC-1 while a C-terminal region, containing a putative cytoplasmic retention signal (Yang et al., Proc. Natl. Acad. Sci. USA 94:13075-13080 (1997);

A cDNA sequence which encodes the full length rat APOBEC-1 is set forth as SEQ ID NO:10 as follows:

The cDNA molecule is reported at Genbank Accession No. L07114,

The full length mouse APOBEC-1 has an ammo acid sequence according to SEQ ID NO: 11 as follows: .

This mouse APOBEC-1 sequence is reported at Genbank Accession No. NP_13112436,-. A cDNA sequence which encodes the full length mouse APOBEC-1 is set forth as SEQ ID NO: 12 as follows: The cDNA molecule is reported at Genbank Accession No. NM_13031159 .

### Signal Sequences

Also disclosed herein are signal sequences, such as secretion sequences, useful in the STS polypeptides and nucleic acids disclosed herein. These sequences can be added to the N-terminal or the C-terminal amino acid sequences. These motifs are present in numerous secreted proteins and have varying abilities to direct a protein during translation to the endoplasmic reticulum and out of the cell. The subject's own cells (hepatocytes, for example) produce and secrete TAT-APOBEC-1 into the local circulation where it can transduce hepatocytes with the liver.

Various signal sequences can be used in the STS polypeptide. One example is an albumin signal sequence. Albumin is a soluble, monomeric protein that is produced by the liver and comprises about one-half of the blood serum protein. Albumin functions primarily as a carrier protein for steroids, fatty acids, and thyroid hormones and plays a role in stabilizing extracellular fluid volume. Mutations in this gene on chromosome 4 result in various anomalous proteins. Albumin is a globular unglycosylated serum protein of molecular weight 65,000. The human albumin gene is 16,961 nucleotides long from the putative 'cap' site to the first poly(A) addition site. It is split into 15 exons which are symmetrically placed within the 3 domains that are thought to have arisen by triplication of a single primordial domain. Albumin is synthesized in the liver as preproalbumin which has an N-terminal peptide that is removed before the nascent protein is released from the rough endoplasmic reticulum. The product, proalbumin, is in turn cleaved in the Golgi vesicles to produce the secreted albumin.

Other signal sequences are known to those of skill in the art, and can be found, for example, in the SIGPEP database. SigPep is a meta-database providing information on prokaryotic and eukaryotic signal peptides found in the public databases. The SigPep database provides information on signal peptide using information like accession number, sequence, and text search. Using relational database as the management system, coupled with a web interface, Sigpep facilitates efficient search of signal peptides. Data is extracted from high quality public databases and automatically updated when these databases are updated. SigPep can be accessed at http://proline.bic.nus.edu.sg/sigpep. Other secretion sequences can be used, which can readily be identified by one of skill in the art. For example, any protein that can be utilized by the liver to secrete TAT-APOBEC can also be used.

### Transduction Sequences

Various transduction sequences can be used in the STS polypeptide. By way of example, protein transduction domains from several known proteins can be employed, including without limitation, HIV-1 Tat protein, Drosophila homeotic transcription factor (ANTP), and HSV-1 VP22 transcription factor (Schwarze et al., TiPS 21:45-48 (2000).

A preferred protein transduction domain is the protein transduction domain of the human immunodeficiency virus ("HIV") Tat protein. An exemplary HIV Tat protein transduction domain has an amino acid sequence of SEQ ID NO:13 as follows:
Arg Lys Lys Arg Arg Gln Arg Arg Arg

This protein transduction domain has also been noted to be a nuclear translocation domain (HIV Sequence Compendium 2000, Kuiken et al. (eds.), Theoretical Biology and Biophysics Group, Los Alamos National Laboratory,). One DNA molecule which encodes the HIV Tat protein transduction domain has a nucleotide sequence of SEQ ID NO:14 as follows:
agaaaaaaaa gaagacaaag aagaaga

Variations of these Tat sequences can also be employed. Such sequence variants have been reported in HIV Sequence Compendium 2000, Kuiken et al. (eds.), Theoretical Biology and Biophysics Group, Los Alamos National Laboratory .

Other cellular uptake polypeptides and their use have been described in the literature, including membrane-permeable sequences of the SN50 peptide, the Grb2 SH2 domain, and integrin β3, β1, and αIIb cytoplasmic domains (Hawiger, Curr. Opin. Chem. Biol. 3:89-94 (1999) .

### Sequence Homology/Identity

It is understood that as discussed herein the use of the terms homology and identity mean the same thing as similarity. Thus, for example, if the use of the word homology is used between two non-natural sequences it is understood that this is not necessarily indicating an evolutionary relationship between these two sequences, but rather is looking at the similarity or relatedness between their nucleic acid sequences. Many of the methods for determining homology between two evolutionarily related molecules are routinely applied to any two or more nucleic acids or proteins for the purpose of measuring sequence similarity regardless of whether they are evolutionarily related or not.

In general, it is understood that one way to define any known variants and derivatives or those that might arise, of the disclosed genes and proteins herein, is through defining the variants and derivatives in terms of homology to specific known sequences. This identity of particular sequences disclosed herein is also discussed elsewhere herein. In general, variants of genes and proteins herein disclosed typically have at least, about 70, 71, 72, 73, 74, 75, 76, 77, 78, 79, 80, 81, 82, 83, 84, 85, 86, 87, 88, 89, 90, 91, 92, 93, 94, 95, 96, 97, 98, or 99 percent homology to the stated sequence or the native sequence. Those of skill in the art readily understand how to determine the homology of two proteins or nucleic acids, such as genes. For example, the homology can be calculated after aligning the two sequences so that the homology is at its highest level.

Another way of calculating homology can be performed by published algorithms. Optimal alignment of sequences for comparison may be conducted by the local homology algorithm of Smith and Waterman Adv. Appl. Math. 2: 482 (1981), by the homology alignment algorithm of Needleman and Wunsch, J. MoL Biol. 48: 443 (1970), by the search for similarity method of Pearson and Lipman, Proc. Natl. Acad. Sci. U.S.A. 85: 2444 (1988), by computerized implementations of these algorithms (GAP, BESTFIT, FASTA, and TFASTA in the Wisconsin Genetics Software Package, Genetics Computer Group, 575 Science Dr., Madison, WI), or by inspection.

The same types of homology can be obtained for nucleic acids by for example the algorithms disclosed in Zuker, M. Science 244:48-52, 1989, Jaeger et al. Proc. Natl. Acad Sci. USA 86:7706-7710, 1989, Jaeger et al. Methods Enzymol. 183:281-306, 1989 . It is understood that any of the methods typically can be used and that in certain instances the results of these various methods may differ, but the skilled artisan understands ifidentity is found with, at least one of these methods, the sequences would be said to have the stated identity, and be disclosed herein.

For example, as used herein, a sequence recited as having a particular percent homology to another sequence refers to sequences that have the recited homology as calculated by any one or more of the calculation methods described above. For example, a first sequence has 80 percent homology, as defined herein, to a second sequence if the first sequence is calculated to have 80 percent homology to the second sequence using the Zuker calculation method even if the first sequence does not have 80 percent homology to the second sequence as calculated by any of the other calculation methods. As another example, a first sequence has 80 percent homology, as defined herein, to a second sequence if the first sequence is calculated to have 80 percent homology to the second sequence using both the Zuker calculation method and the Pearson and Lipman calculation method even if the first sequence does not have 80 percent homology to the second sequence as calculated by the Smith and Waterman calculation method, the Needleman and Wunsch calculation method, the Jaeger calculation methods, or any of the other calculation methods. As yet another example, a first sequence has 80 percent homology, as defined herein, to a second sequence if the first sequence is calculated to have 80 percent homology to the second sequence using each of calculation methods (although, in practice, the different calculation methods will often result in different calculated homology percentages).

The term hybridization typically means a sequence driven interaction between at least two nucleic acid molecules, such as a primer or a probe and a gene. Sequence driven interaction means an interaction that occurs between two nucleotides or nucleotide analogs or nucleotide derivatives in a nucleotide specific manner. For example, G interacting with C or A interacting with T are sequence driven interactions. Typically sequence driven interactions occur on the Watson-Crick face or Hoogsteen face of the nucleotide. The hybridization of two nucleic acids is affected by a number of conditions and parameters known to those of skill in the art. For example, the salt concentrations, pH, and temperature of the reaction all affect whether two nucleic acid molecules will hybridize.

Parameters for selective hybridization between two nucleic acid molecules are well known to those of skill in the art. For example, in some embodiments selective hybridization conditions can be defined as stringent hybridization conditions. For example, stringency of hybridization is controlled by both temperature and salt concentration of either or both of the hybridization and washing steps. For example, the conditions of hybridization to achieve selective hybridization may involve hybridization in high ionic strength solution (6X SSC or 6X SSPE) at a temperature that is about 12-25°C below the Tm (the melting temperature at which half of the molecules dissociate from their hybridization partners) followed by washing at a combination of temperature and salt concentration chosen so that the washing temperature is about 5°C to 20°C below the Tm. The temperature and salt conditions are readily determined empirically in preliminary experiments in which samples of reference DNA immobilized on filters are hybridized to a labeled nucleic acid of interest and then washed under conditions of different stringencies. Hybridization temperatures are typically higher for DNA-RNA and RNA-RNA hybridizations. The conditions can be used as described above to achieve stringency, or as is known in the art. (Sambrook et al., Molecular Cloning: A Laboratory Manual, 2nd Ed., Cold Spring Harbor Laboratory, Cold Spring Harbor, New York, 1989; Kunkel et al. Methods Enzymol. 1987:154:367, 1987 ). A preferable stringent hybridization condition for a DNA:DNA hybridization can be at about 68°C (in aqueous solution) in 6X SSC or 6X SSPE followed by washing at 68°C. Stringency of hybridization and washing, if desired, can be reduced accordingly as the degree of complementarity desired is decreased, and further, depending upon the G-C or A-T richness of any area wherein variability is searched for. Likewise, stringency of hybridization and washing, if desired, can be increased accordingly as homology desired is increased, and further, depending upon the G-C or AT richness of any area wherein high homology is desired, all as known in the art.

Another way to define selective hybridization is by looking at the amount (percentage) of one of the nucleic acids bound to the other nucleic acid. For example, in some embodiments selective hybridization conditions would be when at least about, 60, 65, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 percent of the limiting nucleic acid is bound to the non-limiting nucleic acid. Typically, the non-limiting primer is in for example, 10 or 100 or 1000 fold excess. This type of assay can be performed at under conditions where both the limiting and non-limiting primer are for example, 10 fold or 100 fold or 1000 fold below their k_{d}, or where only one of the nucleic acid molecules is 10 fold or 100 fold or 1000 fold or where one or both nucleic acid molecules are above their k_{d}.

Another way to define selective hybridization is by looking at the percentage of primer that gets enzymatically manipulated under conditions where hybridization is required to promote the desired enzymatic manipulation. For example, in some embodiments selective hybridization conditions would be when at least about, 60, 65, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 percent of the primer is enzymatically manipulated under conditions which promote the enzymatic manipulation, for example if the enzymatic manipulation is DNA extension, then selective hybridization conditions would be when at least about 60, 65, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 percent of the primer molecules are extended. Preferred conditions also include those suggested by the manufacturer or indicated in the art as being appropriate for the enzyme performing the manipulation.

Just as with homology, it is understood that there are a variety of methods herein disclosed for determining the level of hybridization between two nucleic acid molecules. It is understood that these methods and conditions may provide different percentages of hybridization between two nucleic acid molecules, but unless otherwise indicated meeting the parameters of any of the methods would be sufficient. For example if 80% hybridization was required and as long as hybridization occurs within the required parameters in any one of these methods it is considered disclosed herein.

It is understood that those of skill in the art understand that if a composition or method meets any one of these criteria for determining hybridization either collectively or singly it is a composition or method that is disclosed herein.

There are a variety of molecules disclosed herein that are nucleic acid based, including for example the nucleic acids that encode the STS polypeptides. The disclosed nucleic acids are made up of for example, nucleotides, nucleotide analogs, or nucleotide substitutes. Non-limiting -examples of these and other molecules are discussed herein. It is understood that for example, when a vector is expressed in a cell, the expressed mRNA will typically be made up of A, C, G, and U. Likewise, it is understood that if, for example, an antisense molecule is introduced into a cell or cell environment through for example exogenous delivery, it is advantageous that the antisense molecule be made up of nucleotide analogs that reduce the degradation of the antisense molecule in the cellular environment.

A nucleotide is a molecule that contains a base moiety, a sugar moiety and a phosphate moiety. Nucleotides can be linked together through their phosphate moieties and sugar moieties creating an internucleoside linkage. The base moiety of a nucleotide can be adenin-9-yl (A), cytosin-1-yl (C), guanin-9-yl (G), uracil-1-yl (U), and thymin-1-yl (T). The sugar moiety of a nucleotide is a ribose or a deoxyribose. The phosphate moiety of a nucleotide is pentavalent phosphate. An non-limiting example of a nucleotide would be 3'-AMP (3'-adenosine monophosphate) or 5'-GMP (5'-guanosine monophosphate).

A nucleotide analog is a nucleotide which contains some type of modification to either the base, sugar, or phosphate moieties. Modifications to nucleotides are well known in the art and would include for example, 5-methylcytosine (5-me-C), 5-hydroxymethyl cytosine, xanthine, hypoxanthine, and 2-aminoadenine as well as modifications at the sugar or phosphate moieties.

Nucleotide substitutes are molecules having similar functional properties to nucleotides, but which do not contain a phosphate moiety, such as peptide nucleic acid (PNA). Nucleotide substitutes are molecules that will recognize nucleic acids in a Watson-Crick or Hoogsteen manner, but which are linked together through a moiety other than a phosphate moiety. Nucleotide substitutes are able to conform to a double helix type structure when interacting with the appropriate target nucleic acid.

It is also possible to link other types of molecules (conjugates) to nucleotides or nucleotide analogs to enhance for example, cellular uptake. Conjugates can be chemically linked to the nucleotide or nucleotide analogs. Such conjugates include but are not limited to lipid moieties such as a cholesterol moiety. (Letsinger et al., Proc. Natl. Acad. Sci. USA, 1989,86, 6553-6556).

A Watson-Crick interaction is at least one interaction with the Watson-Crick face of a nucleotide, nucleotide analog, or nucleotide substitute. The Watson-Crick face of a nucleotide, nucleotide analog, or nucleotide substitute includes the C2, N1, and C6 positions of a purine based nucleotide, nucleotide analog, or nucleotide substitute and the C2, N3, C4 positions of a pyrimidine based nucleotide, nucleotide analog, or nucleotide substitute.

A Hoogsteen interaction is the interaction that takes place on the Hoogsteen face of a nucleotide or nucleotide analog, which is exposed in the major groove of duplex DNA. The Hoogsteen face includes the N7 position and reactive groups (NH2 or O) at the C6 position of purine nucleotides.

### Sequences

There are a variety of sequences related to, for example, STS polypeptides, as well as any other protein disclosed herein that are disclosed on Genbank . The disclosed sequences can be naturally occurring sequences or fragments thereof, variants of sequences or fragments thereof, or modified forms of sequences or fragments thereof. Thus, the disclosed APOBEC-1 sequences can be naturally occurring APOBEC-1 sequences or fragments thereof or fragments thereof having RNA editing activity, variants of narurally occurring APOBEC-1 sequences or fragments thereof or fragments thereof having RNA editing activity, modified forms of narurally occurring APOBEC-1 sequences or fragments thereof or fragments thereof having RNA editing activity. Similarly, the disclosed secretion sequences can be naturally occurring secretion sequences or fragments thereof or fragments thereof having secretion activity, variants of narurally occurring secretion sequences or fragments thereof or fragments thereof having secretion activity, modified forms of narurally occurring secretion sequences or fragments thereof or fragments thereof having secretion activity. The disclosed transduction sequences can be naturally occurring transduction sequences or fragments thereof or fragments thereof having tranduction activity, variants of narurally occurring transduction sequences or fragments thereof or fragments thereof having transduction activity, modified forms of narurally occurring transduction sequences or fragments thereof or fragments thereof having transduction activity. The disclosed STS sequences and polypeptides can comprise such varinats, modified forms, and fragments. Variants, modifications and fragments of sequences are described below and elsewhere herein.

A variety of sequences are provided herein and these and others can be found in Genbank, at www.pubmed.gov. Those of skill in the art understand how to resolve sequence discrepancies and differences and to adjust the compositions and methods relating to a particular sequence to other related sequences. Primers and/or probes can be designed for any sequence given the information disclosed herein and known in the art.

Disclosed are compositions including primers and probes, which are capable of interacting with the nucleic acids disclosed herein. In certain embodiments the primers are used to support DNA amplification reactions. Typically the primers will be capable of being extended in a sequence specific manner. Extension of a primer in a sequence specific manner includes any methods wherein the sequence and/or composition of the nucleic acid molecule to which the primer is hybridized or otherwise associated directs or influences the composition or sequence of the product produced by the extension of the primer. Extension of the primer in a sequence specific manner therefore includes, but is not limited to, PCR, DNA sequencing, DNA extension, DNA polymerization, RNA transcription, or reverse transcription. Techniques and conditions that amplify the primer in a sequence specific manner are preferred. In certain embodiments the primers are used for the DNA amplification reactions, such as PCR or direct sequencing. It is understood that in certain embodiments the primers can also be extended using non-enzymatic techniques, where for example, the nucleotides or oligonucleotides used to extend the primer are modified such that they will chemically react to extend the primer in a sequence specific manner. Typically the disclosed primers hybridize with the nucleic acid or region of the nucleic acid or they hybridize with the complement of the nucleic acid or complement of a region of the nucleic acid.

Disclosed are isolated nucleic acid sequences comprising a sequence at least 80% identical to SEQ ID NOs:1-5, 8, 10, 12, or 14. The nucleic acid sequence comprising the nucleotide sequence of SEQ ID NOs:1-5, 8, 10, 12, and 14, or variants or fragments thereof, wherein the variant or fragment comprises a specific sequence. Also contemplated are isolated nucleic acid sequences comprising at least five consecutive nucleotides of SEQ ID NOS: 1-5, 8, 10, 12, or 14 wherein the nucleic acid sequence comprises a specific sequence. Also contemplated are isolated nucleic acid sequences comprising the sequence of SEQ ID NOS: 1-5, 8, 10, 12, or 14.

In the methods disclosed herein, molecules such as STS polypeptides can be used in assays. These molecules can be, for example, chimeric proteins. By "chimeric protein" is meant any single polypeptide unit that comprises two distinct polypeptide domains joined by a peptide bond, optionally by means of an amino acid linker, or a non-peptide bond, wherein the two domains are not naturally occurring within the same polypeptide unit. Typically, such chimeric proteins are made by expression of a nucleic acid construct but could be made by protein synthesis methods known in the art. These chimeric proteins are useful in screening compounds, as well as with the compounds identified by the methods disclosed herein. The STS polypeptide itself is considered a chimeric protein, as it comprises an APOBEC-1 polypeptide, a transduction sequence, and a signal sequence.

The compositions disclosed herein can also be fragments or derivatives of a naturally occurring deaminase or viral infectivity factor. A "fragment" is a polypeptide that is less than the full length of a particular protein or functional domain. By "derivative" or "variant" is meant a polypeptide having a particular sequence that differs at one or more positions from a reference sequence. The fragments or derivatives of a full length protein preferably retain at least one function of the full length protein. For example, a fragment or derivative of an STS polypeptide includes a fragment of APOBEC-1 that retains at least one function of the full length protein, such as its editing ability. The fragment or derivative can include conservative or non-conservative amino acid substitutions. The fragment or derivative optionally can form a homodimer or a homotetramer.

Protein variants and derivatives are well understood to those of skill in the art and can involve amino acid sequence modifications. For example, amino acid sequence modifications typically fall into one or more of three classes: substitutional, insertional or deletional variants. Insertions-include amino and/or carboxyl-terminal fusions as well as intrasequence insertions of single or multiple amino acid residues. Insertions ordinarily will be smaller insertions than those of amino or carboxyl terminal fusions, for example, on the order of one to four residues. Immunogenic fusion protein derivatives, such as those described in the examples, are made by fusing a polypeptide sufficiently large to confer immunogenicity to the target sequence by crosslinking in vitro or by recombinant cell culture transformed with DNA encoding the fusion. Deletions are characterized by the removal of one or more amino acid residues from the protein sequence. Typically, no more than about from 2 to 6 residues are deleted at any one site within the protein molecule. These variants ordinarily are prepared by site specific mutagenesis of nucleotides in the DNA encoding the protein, thereby producing DNA encoding the variant, and thereafter expressing the DNA in recombinant cell culture. Techniques for making substitution mutations at predetermined sites in DNA having a known sequence are well known, for example M13 primer mutagenesis and PCR mutagenesis. Amino acid substitutions are typically of single residues, but can occur at a number of different locations at once; insertions usually will be on the order of about from 1 to 10 amino acid residues; and deletions will range about from 1 to 30 residues. Deletions or insertions preferably are made in adjacent pairs, i.e. a deletion of 2 residues or insertion of 2 residues. Substitutions, deletions, insertions or any combination thereof may be combined to arrive at a final construct. The mutations must not place the sequence out of reading frame and preferably will not create complementary regions that could produce secondary mRNA structure. Substitutional variants are those in which at least one residue has been removed and a different residue inserted in its place. Such substitutions generally are made in accordance with the following Table 2 and are referred to as conservative substitutions.

Substantial changes in function or immunological identity are made by selecting substitutions that are less conservative, i.e., selecting residues that differ more significantly in their effect on maintaining (a) the structure of the polypeptide backbone in the area of the substitution, for example as a sheet or helical confirmation, (b) the charge or hydrophobicity of the molecule at the target site or (c) the bulk of the side chain. The substitutions which in general are expected to produce the greatest changes in the protein properties will be those in which (a) a hydrophilic residue, e.g. seryl or threonyl, is substituted for (or by) a hydrophobic residue, e.g. leucyl, isoleucyl, phenylalanyl, valyl or alanyl; (b) a cysteine or proline is substituted for (or by) any other residue; (c) a residue having an electropositive side chain, e.g., lysyl, arginyl, or histidyl, is substituted for (or by) an electronegative residue, e.g., glutamyl or aspartyl; or (d) a residue having a bulky side chain, e.g., phenylalanine, is substituted for (or by) one not having a side chain, e.g., glycine, in this case, (e) by increasing the number of sites for sulfation and/or glycosylation.

**TABLE 2: Amino Acid Substitutions**

| **Original Residue** | **Exemplary Substitutions** |
|---|---|
| Ala | Ser |
| Arg | Lys |
| Asn | Gln |
| Asp | Glu |
| Cys | Ser |
| Gln | Asn |
| Glu | Asp |
| Gly | Pro |
| His | Gln |
| Ile | Leu; Val |
| Leu | Ile; Val |
| Lys | Arg; Gln |
| Met | Leu; Ile |
| Phe | Met; Leu; Tyr |
| Pro | Gly |
| Ser | Thr |
| Thr | Ser |
| Trp | Tyr |
| Tyr | Trp; Phe |
| Val | Ile; Leu |

For example, the replacement of one amino acid residue with another that is biologically and/or chemically similar is known to those skilled in the art as a conservative substitution. For example, a conservative substitution would be replacing one hydrophobic residue for another, or one polar residue for another. The substitutions include combinations such as, for example, Gly, Ala; Val, Ile, Leu; Asp, Glu; Asn, Gln; Ser, Thr; Lys, Arg; and Phe, Tyr. Such conservatively substituted variations of each explicitly disclosed sequence are included within the mosaic polypeptides provided herein.

Substitutional or deletional mutagenesis can be employed to insert sites for N-glycosylation (Asn-X-Thr/Ser) or O-glycosylation (Ser or Thr). Deletions of cysteine or other labile residues also may be desirable. Deletions or substitutions of potential proteolysis sites, e.g. Arg, is accomplished for example by deleting one of the basic residues or substituting one by glutaminyl or histidyl residues.

Certain post-translational derivatizations are the result of the action of recombinant host cells on the expressed polypeptide. Glutaminyl and asparaginyl residues are frequently post-translationally deamidated to the corresponding glutamyl and asparyl residues. Alternatively, these residues are deamidated under mildly acidic conditions. Other post-translational modifications include hydroxylation of proline and lysine, phosphorylation of hydroxyl groups of seryl or threonyl residues, methylation of the o-amino groups of lysine,-arginine, and histidine side chains (T.E. Creighton, Proteins: Structure and Molecular Properties, W. H. Freeman & Co., San Francisco pp 79-86 [1983]), acetylation of the N-terminal amine and, in some instances, amidation of the C-terminal carboxyl.

### Delivery of the compositions to cells

There are a number of compositions and methods which can be used to deliver nucleic acids to cells, either *in vitro* or *in vivo*. These methods and compositions can largely be broken down into two classes: viral based delivery systems and non-viral based delivery systems. For example, the nucleic acids can be delivered through a number of direct delivery systems such as electroporation, lipofection, calcium phosphate precipitation, plasmids, viral vectors, viral nucleic acids, phage nucleic acids, phages, cosmids, or via transfer of genetic material in cells or carriers such as cationic liposomes. Appropriate means for transfection, including viral vectors, chemical transfectants, or physico-mechanical-methods such as electroporation and direct diffusion of DNA, are described by, for example, Wolff, J. A., et al., Science, 247, 1465-1468, (1990); and Wolff, J. A. Nature, 352, 815-818, (1991). Such methods are well known in the art and readily adaptable for use with the compositions and methods described herein. In certain cases, the methods will be modified to specifically function with large DNA molecules. Further, these methods can be used to target certain diseases and cell populations by using the targeting characteristics of the carrier.

### Nucleic acid based delivery systems

Transfer vectors can be any nucleotide construction used to deliver genes into cells (e.g., a plasmid), or as part of a general strategy to deliver genes, e.g., as part of recombinant retrovirus or adenovirus (Ram et al. Cancer Res. 53:83-88, (1993)).

As used herein, plasmid or viral vectors are agents that transport the disclosed nucleic acids, such as those encoding an STS polypeptide, into the cell without degradation and include a promoter yielding expression of the-gene in the cells into which it is delivered. In some embodiments the vectors are derived from either a virus or a retrovirus. Viral vectors are, for example, Adenovirus, Adeno-associated virus, Herpes virus, Vaccinia virus, Polio virus, AIDS virus, neuronal trophic virus, Sindbis and other RNA viruses, including these viruses with the HIV backbone. Also preferred are any viral families which share the properties of these viruses which make them suitable for use as vectors. Retroviruses include Murine Maloney Leukemia virus, MMLV, and retroviruses that express the desirable properties of MMLV as a vector. Retroviral vectors are able to carry a larger-genetic payload, i.e., a transgene or marker gene, than other viral vectors, and for this reason are a commonly used vector. However, they are not as useful in non-proliferating cells. Adenovirus vectors are relatively stable and easy to work with, have high titers, and can be delivered in aerosol formulation, and can transfect non-dividing cells. Pox viral vectors are large and have several sites for inserting genes, they are thermostable and can be stored at room temperature. A preferred embodiment is a viral vector which has been engineered so as to suppress the immune response of the host organism, elicited by the viral antigens. Preferred vectors of this type will carry coding regions for Interleukin 8 or 10.

Viral vectors can have higher transaction (ability to introduce genes) abilities than chemical or physical methods to introduce genes into cells. Typically, viral vectors contain, nonstructural early genes, structural late genes, an RNA polymerase III transcript, inverted terminal repeats necessary for replication and encapsidation, and promoters to control the transcription and replication of the viral genome. When engineered as vectors, viruses typically have one or more of the early genes removed and a gene or gene/promoter cassette is inserted into the viral genome in place of the removed viral DNA. Constructs of this type can carry up to about 8 kb of foreign genetic material. The necessary functions of the removed early genes are typically supplied by cell lines which have been engineered to express the gene products of the early genes in *trans.*

### Adenoviral Vectors

The construction of replication-defective adenoviruses has been described (Berkner et al., J. Virology 61:1213-1220 (1987); Massie et al., Mol. Cell. Biol. 6:2872-2883 (1986); Haj-Ahmad et al., J. Virology 57:267-274 (1986); Davidson et al., J. Virology 61:1226-1239 (1987); Zhang "Generation and identification of recombinant adenovirus by liposome-mediated transfection and PCR analysis" BioTechniques 15:868-872 (1993)). The benefit of the use of these viruses as vectors is that they are limited in the extent to which they can spread to other cell types, since they can replicate within an initial infected cell, but are unable to form new infectious viral particles. Recombinant adenoviruses have been shown to achieve high efficiency gene transfer after direct, in vivo delivery to airway epithelium, hepatocytes, vascular endothelium, CNS parenchyma and a number of other tissue sites (Morsy, J. Clin. Invest. 92:1580-1586 (1993); Kirshenbaum, J. Clin. Invest. 92:381-387 (1993); Roessler, J. Clin. Invest. 92:1085-1092 (1993); Moullier, Nature Genetics 4:154-159 (1993); La Salle, Science 259:988-990 (1993); Gomez-Foix, J. Biol. Chem. 267:25129-25134 (1992); Rich, Human Gene Therapy 4:461-476 (1993); Zabner, Nature Genetics 6:75-83 (1994); Guzman, Circulation Research 73:1201-1207 (1993); Bout, Human Gene Therapy 5:3-10 (1994); Zabner, Cell 75:207-216 (1993); Caillaud, Eur. J. Neuroscience 5:1287-1291 (1993); and Ragot, J. Gen. Virology 74:501-507 (1993)). Recombinant adenoviruses achieve gene transduction by-binding to-specific cell surface receptors, after which the virus is internalized by receptor-mediated endocytosis, in the same manner as wild type or replication-defective adenovirus (Chardonnet and Dales, Virology 40:462-477 (1970); Brown and Burlingham, J. Virology 12:386-396 (1973); Svensson and Persson, J. Virology 55:442-449 (1985); Seth, et al., J. Virol. 51:650-655 (1984); Seth, et al., Mol. Cell. Biol. 4:1528-1533 (1984); Varga et al., J. Virology 65:6061-6070 (1991); Wickham et al., Cell 73:309-319 (1993)).

A viral vector can be one based on an adenovirus which has had the E1 gene removed and these virons are generated in a cell line such as the human 293 cell line. In another preferred embodiment both the E1 and E3 genes are removed from the adenovirus genome.

### Adeno-associated viral vectors

Another type of viral vector is based on an adeno-associated virus (AAV). This defective parvovirus is a preferred vector because it can infect many cell types and is nonpathogenic to humans. AAV type vectors can transport about 4 to 5 kb and wild type AAV is known to stably insert into chromosome 19. Vectors which contain this site specific integration property are preferred. An especially preferred embodiment of this type of vector is the P4.1 C vector produced by Avigen, San Francisco, CA, which can contain the herpes simplex virus thymidine kinase gene, HSV-tk, and/or a marker gene, such as the gene encoding the green fluorescent protein, GFP.

In another type of AAV virus, the AAV contains a pair of inverted terminal repeats (ITRs) which flank at least one cassette containing a promoter which directs cell-specific expression operably linked to a heterologous gene. Heterologous in this context refers to any nucleotide sequence or gene which is not native to the AAV or B19 parvovirus.

Typically the AAV and B 19 coding regions have been deleted, resulting in a safe, noncytotoxic vector. The AAV ITRs, or modifications thereof, confer infectivity and site-specific integration, but not cytotoxicity, and the promoter directs cell-specific expression: (United states Patent No. 6,261,834).

The disclosed vectors thus provide DNA molecules which are capable of integration into a mammalian chromosome without substantial toxicity.

The inserted genes in viral and retroviral usually contain promoters, and/or enhancers to help control the expression of the desired gene product. A promoter is generally a sequence or sequences of DNA that function when in a relatively fixed location in regard to the transcription start site. A promoter contains core elements required for basic interaction of RNA polymerase and transcription factors, and may contain upstream elements and response elements.

### Large payload viral vectors

Molecular genetic experiments with large human herpes viruses have provided a means whereby large heterologous DNA fragments can be cloned, propagated and established in cells permissive for infection with herpes viruses (Sun et al., Nature genetics 8: 33-41, 1994; Cotter and Robertson, Cun Opin Mol Ther 5: 633-644, 1999). These large DNA viruses (herpes simplex virus (HSV) and Epstein-Barr virus (EBV), have the potential to deliver fragments of human heterologous DNA > 150 kb to specific cells. EBV recombinants can maintain large pieces of DNA in the infected B-cells as episomal DNA. Individual clones carried human genomic inserts up to 330 kb appeared genetically stable. The maintenance of these episomes requires a specific EBV nuclear protein, EBNA1, constitutively expressed during infection with EBV. Additionally, these vectors can be used for transfection, where large amounts of protein can be generated transiently *in vitro.* Herpes virus amplicon systems are also being used to package pieces of DNA > 220 kb and to infect cells that can stably maintain DNA as episomes.

Other useful systems include, for example, replicating and host-restricted non-replicating vaccinia virus vectors.

In the methods described above which include the administration and uptake of exogenous DNA into the cells of a subject (i.e., gene transduction or transfection), delivery of the compositions to cells can be via a variety of mechanisms. As one example, delivery can be via a liposome, using commercially available liposome preparations such as LIPOFECTIN, LIPOFECTAMINE (GIBCO-BRL, Inc., Gaithersburg, MD), SUPERFECT (Qiagen, Inc. Hilden, Germany) and TRANSFECTAM (Promega Biotec, Inc., Madison, WI), as well as other liposomes developed according to procedures standard in the art. In addition, the disclosed nucleic acid or vector can be delivered in vivo by electroporation, the technology for which is available from Genetronics, Inc. (San Diego, CA) as well as by means of a SONOPORATION machine (ImaRx Pharmaceutical Corp., Tucson, AZ).

The materials may be in solution, suspension (for example, incorporated into microparticles, liposomes, or cells). These may be targeted to a particular cell type via antibodies, receptors, or receptor ligands. The following references are examples of the use of this technology to target specific proteins to tumor tissue (Senter, et al., Bioconjugate Chem., 2:447-451, (1991); Bagshawe, K.D., Br. J. Cancer, 60:275-281, (1989); Bagshawe, et al., Br. J. Cancer, 58:700-703, (1988); Senter, et al., Bioconjugate Chem., 4:3-9, (1993); Battelli, et al., Cancer Immunol. Immunother., 35:421-425, (1992); Pietersz and McKenzie, Immunolog. Reviews, 129:57-80, (1992); and Roffler, et al., Biochem. Pharmacol, 42:2062-2065, (1991)). These techniques can be used for a variety of other specific cell types. Vehicles such as "stealth" and other antibody conjugated liposomes (including lipid mediated drug targeting to colonic carcinoma), receptor mediated targeting of DNA through cell specific ligands, lymphocyte directed tumor targeting, and highly specific therapeutic retroviral targeting of murine glioma cells in vivo. The following references are examples of the use of this technology to target specific proteins to tumor tissue (Hughes et al., Cancer Research, 49:62.14-6220, (1989); and Litzinger and Huang, Biochimica et Biophysica Acta, 1104:179-187, (1992)). In general, receptors are involved in pathways of endocytosis, either constitutive or ligand induced. These receptors cluster in clathrin-coated pits, enter the cell via clathrin-coated vesicles, pass through an acidified endosome in which the receptors are sorted, and then either recycle to the cell surface, become stored intracellularly, or are degraded in lysosomes. The internalization pathways serve a variety of functions, such as nutrient uptake, removal of activated proteins, clearance of macromolecules, opportunistic entry of viruses and toxins, dissociation and degradation of ligand, and receptor-level regulation. Many receptors follow more than one intracellular pathway, depending on the cell type, receptor concentration, type of ligand, ligand valency, and ligand concentration. Molecular and cellular mechanisms of receptor-mediated endocytosis has been reviewed (Brown and Greene, DNA and Cell Biology 10:6, 399-409 (1991)).

Nucleic acids that are delivered to cells which are to be integrated into the host cell genome typically contain integration sequences. These sequences are often viral related sequences, particularly when viral based systems are used. These viral integration systems can also be incorporated into nucleic acids which are to be delivered using a non-nucleic acid based system of deliver, such as a liposome, so that the nucleic acid contained in the delivery system can be come integrated into the host genome.

Other general techniques for integration into the host genome include, for example, systems designed to promote homologous recombination with the host genome. These systems typically rely on sequence flanking the nucleic acid to be expressed that has enough homology with a target sequence within the host cell genome that recombination between the vector nucleic acid and the target nucleic acid takes place, causing the delivered nucleic acid to be integrated into the host genome. These systems and the methods necessary to promote homologous recombination are known to those of skill in the art.

### In vivo/ex vivo

As described above, the STS nucleic acids can be administered in a pharmaceutically acceptable carrier and can be delivered to the subject's cells *in vivo* and/or *ex vivo* by a variety of mechanisms well known in the art (e.g., uptake of naked DNA, liposome fusion, intramuscular injection of DNA via a gene gun, endocytosis and the like).

If *ex vivo* methods are employed, cells or tissues can be removed and maintained outside the body according to standard protocols well known in the art. The compositions can be introduced into the cells via any gene transfer mechanism, such as, for example, calcium phosphate mediated gene delivery, electroporation, microinjection or proteoliposomes. The transduced cells can then be infused (e.g., in a pharmaceutically acceptable carrier) or homotopically transplanted back into the subject per standard methods for the cell or tissue type. Standard methods are known for transplantation or infusion of various cells into a subject.

### Expression systems

The nucleic acids that are delivered to cells typically contain expression controlling systems. For example, the inserted genes in viral and retroviral systems usually contain promoters, and/or enhancers to help control the expression of the desired gene product. A promoter is generally a sequence or sequences of DNA that function when in a relatively fixed location in regard to the transcription start site. A promoter contains core elements required for basic interaction of RNA polymerase and transcription factors, and may contain upstream elements and response elements.

### Viral Promoters and Enhancers

Preferred promoters controlling transcription from vectors in mammalian host cells may be obtained from various sources, for example, the genomes of viruses such as: polyoma, Simian Virus 40 (SV40), adenovirus, retroviruses, hepatitis-B virus and most preferably cytomegalovirus, or from heterologous mammalian promoters, e.g. beta actin promoter. The early and late promoters of the SV40 virus are conveniently obtained as an SV40 restriction fragment which also contains the SV40 viral origin of replication (Fiers et al., Nature, 273: 113 (1978)). The immediate early promoter of the human cytomegalovirus is conveniently obtained as a HindIII E restriction fragment (Greenway, P.J. et al., Gene 18: 355-360 (1982)). Of course, promoters from the host cell or related species also are useful herein.

Enhancer generally refers to a sequence of DNA that functions at no fixed distance from the transcription start site and can be either 5' (Laimins, L. et al., Proc. Natl. Acad. Sci. 78: 993 (1981)) or 3' (Lusky, M.L., et al., Mol. Cell Bio. 3: 1108 (1983)) to the transcription unit. Furthermore, enhancers can be within an intron (Banerji, J.L. et al., Cell 33: 729 (1983)) as well as within the coding sequence itself (Osborne, T.F., et al., Mol. Cell Bio. 4: 1293 (1984)). They are usually between 10 and 300 bp in length, and they function in cis. Enhancers function to increase transcription from nearby promoters. Enhancers also often contain response elements that mediate the regulation of transcription. Promoters can also contain response elements that mediate the regulation of transcription. Enhancers often determine the regulation of expression of a gene. While many enhancer sequences are now known from mammalian genes (globin, elastase, albumin, fetoprotein and insulin), typically one will use an enhancer from a eukaryotic cell virus for general expression. Preferred examples are the SV40 enhancer on the late side of the replication origin (bp 100-270), the cytomegalovirus early promoter enhancer, the polyoma enhancer on the late side of the replication origin, and adenovirus enhancers.

The promoter and/or enhancer may be specifically activated either by light or specific chemical events which trigger their function. Systems can be regulated by reagents such as tetracycline and dexamethasone. There are also ways to enhance viral vector gene expression by exposure to irradiation, such as gamma irradiation, or alkylating chemotherapy drugs.

In certain embodiments the promoter and/or enhancer region can act as a constitutive promoter and/or enhancer to maximize expression of the region of the transcription unit to be transcribed. In certain constructs the promoter and/or enhancer region be active in all eukaryotic cell types, even if it is only expressed in a particular type of cell at a particular time. A preferred promoter of this type is the CMV promoter (650 bases). Other preferred promoters are SV40 promoters, cytomegalovirus (full length promoter), and retroviral vector LTR.

It has been shown that all specific regulatory elements can be cloned and used to construct expression vectors that are selectively expressed in specific cell types such as melanoma cells. The glial fibrillary acetic protein (GFAP) promoter has been used to selectively express genes in cells of glial origin.

Expression vectors used in eukaryotic host cells (yeast, fungi, insect, plant, animal, human or nucleated cells) can also contain sequences necessary for the termination of transcription which may affect mRNA expression. These regions are transcribed as polyadenylated segments in the untranslated portion of the mRNA encoding tissue factor protein. The 3' untranslated regions can also include transcription termination sites. It is preferred that the transcription unit also contain a polyadenylation region. One benefit of this region is that it increases the likelihood that the transcribed unit will be processed and transported like mRNA. The identification and use of polyadenylation signals in expression constructs is well established. It is preferred that homologous polyadenylation signals be used in the transgene constructs. In certain transcription units, the polyadenylation region is derived from the SV40 early polyadenylation signal and consists of about 400 bases. It is also preferred that the transcribed units contain other standard sequences alone or in combination with the above sequences improve expression from, or stability of, the construct.

### Markers

The viral vectors can include nucleic acid sequence encoding a marker product. This marker product can be used to determine if the gene has been delivered to the cell and once delivered is being expressed. Preferred marker genes are the E. Coli lacZ gene, which encodes β-galactosidase, and green fluorescent protein.

In some embodiments the marker may be a selectable marker. Examples of suitable selectable markers for mammalian cells are dihydrofolate reductase (DHFR), thymidine kinase, neomycin, neomycin analog G418, hydromycin, and puromycin. When such selectable markers are successfully transferred into a mammalian host cell, the transformed mammalian host cell can survive if placed under selective pressure. There are two widely used distinct categories of selective regimes. The first category is based on a cell's metabolism and the use of a mutant cell line which lacks the ability to grow independent of a supplemented media. Two examples are: CHO DHFR cells and mouse LTK- cells. These cells lack the ability to grow without the addition of such nutrients as thymidine or hypoxanthine. Because these cells lack certain genes necessary for a complete nucleotide synthesis pathway, they cannot survive unless the missing nucleotides are provided in a supplemented media. An alternative to supplementing the media is to introduce an intact DHFR or TK gene into cells lacking the respective genes, thus altering their growth requirements. Individual cells which were not transformed with the DHFR or TK gene will not be capable of survival in non-supplemented media.

The second category is dominant selection which refers to a selection scheme used in any cell type and does not require the use of a mutant cell line. These schemes typically use a drug to arrest growth of a host cell. Those cells which have a novel gene would express a protein conveying drug resistance and would survive the selection. Examples of such dominant selection use the drugs neomycin, (Southern P. and Berg, P., J. Molec. Appl. Genet. 1:327 (1982)), mycophenolic acid, (Mulligan, R.C. and Berg, P. Science 209: 1422 (1980)) or hygromycin, (Sugden, B. et al., Mol. Cell. Biol. 5: 410-413 (1985)). The three examples employ bacterial genes under eukaryotic control to convey resistance to the appropriate drug G418 or neomycin (geneticin), xgpt (mycophenolic acid) or hygromycin, respectively. Others include the neomycin analog G418 and puramycin.

### Therapeutic Uses

Effective dosages and schedules for administering the compositions, such as STS nucleic acids, may be determined empirically, and making such determinations is within the skill in the art. The dosage ranges for the administration of the compositions are those large enough to produce the desired effect in which the symptoms or disorder are effected. The dosage should not be so large as to cause adverse side effects, such as unwanted cross-reactions, anaphylactie reactions, and the like. Generally, the dosage will vary with the age, condition, sex and extent of the disease in the patient, route of administration, or whether other drugs are included in the regimen, and can be determined by one of skill in the art. The dosage can be adjusted by the individual physician in the event of any contraindications. Dosage can vary, and can be administered in one or more dose administrations daily, for one or several days. Guidance can be found in the literature for appropriate dosages for given classes of nucleic acids. A typical daily dosage of the vectors disclosed herein might range from about 1 µg/kg to up to 100 mg/kg of body weight or more per day, depending on the factors mentioned above. For example, vector dosage can be given according to Naki et al., (J. Virol., 1999, 73:5438-47). In one example, the dosage can be 5 x 10⁹, 5 x 10¹⁰ or 5 x 10¹¹ gene copies (GC)/injection/subject.

Following administration of a disclosed composition, such as an STS nucleic acid, for treating, inhibiting, or preventing diseases or disorders associated with ApoB100, the efficacy of the therapeutic nucleic acid can be assessed in various ways well known to the skilled practitioner. For instance, one of ordinary skill in the art will understand that a composition, such as a nucleic acid, disclosed herein is efficacious in reducing atherogenic risk in a subject by observing that the composition reduces the level of ApoB100, for example.

The compositions that reduce the level of ApoH100 disclosed herein may be administered prophylactically to patients or subjects who are at risk for atherogenesis, or who have experienced dyslipidemia or cardiovascular disease. In subjects who are at risk for atherogenesis but who have not yet experienced symptoms, efficacious treatment with an STS nucleic acid or polypeptide can partially or completely inhibit the appearance of atherogenesis.

The disclosed compositions and methods can be used in combination with other compositions and methods. For example, there are many compositions and treatments that are available for treating the disorders and diseases disclosed herein related to ApoB100. Examples include statin drugs. Statins are drugs that improve cholesterol levels primarily by inhibiting the liver enzyme HMG C-A reductase.Statins have proven to be very effective in reducing cholesterol and in reducing the risk of heart attack and death. Statins include, but are not limited to, atorvastatin (Lipitor), fluvastatin (Lescol), lovastatin (Mevacor), pravastatin (Pravachol), simvastatin (Zocor), and rosuvastatin (Crestor).

The methods disclosed herein can also be used in combination with lipid-lowering bile-acid-binding resin therapy, which has shown efficacy via sequestering the bile acids in the intestine, thereby interrupting the enterohepatic circulation of bile acids and increasing the elimination of cholesterol from the body.

Other molecules that interact with ApoB100 which do not have a specific pharmaceutical function, but which may be used for tracking changes within cellular chromosomes or for the delivery of diagnostic tools for example can be delivered in ways similar to those described for the pharmaceutical products.

The disclosed compositions and methods can also be used for example as tools to isolate and test new drug candidates for a variety of ApoB100- related diseases.

### Chips and micro arrays

Disclosed are chips where at least one address is the sequences or part of the sequences set forth in any of the nucleic acid sequences disclosed herein. Also disclosed are chips where at least one address is the sequences or portion of sequences set forth in any of the peptide sequences disclosed herein.

Also disclosed are chips where at least one address is a variant of the sequences or part of the sequences set forth in any of the nucleic acid sequences disclosed herein. Also disclosed are chips where at least one address is a variant of the sequences or portion of sequences set forth in any of the peptide sequences disclosed herein.

### Computer readable mediums

It is understood that the disclosed nucleic acids and proteins can be represented as a sequence consisting of the nucleotides of amino acids. There are a variety of ways to display these sequences, for example the nucleotide guanosine can be represented by G or g. Likewise the amino acid valine can be represented by Val or V. Those of skill in the art understand how to display and express any nucleic acid or protein sequence in any of the variety of ways that exist, each of which is considered herein disclosed. Specifically contemplated herein is the display of these sequences on computer readable mediums, such as, commercially available floppy disks, tapes, chips, hard drives, compact disks, and video disks, or other computer readable mediums. Also disclosed are the binary code representations of the disclosed sequences. Those of skill in the art understand what computer readable mediums. Thus, computer readable mediums on which the nucleic acids or protein sequences are recorded, stored, or saved.

### Compositions identified by screening with disclosed composition/ combinatorial chemistry

The disclosed compositions can be used as targets for any combinatorial technique to identify molecules or macromolecular molecules that interact with the disclosed compositions in a desired way. The nucleic acids, peptides, and related molecules disclosed herein can be used as targets for the combinatorial approaches. Also disclosed are the compositions that are identified through combinatorial techniques or screening techniques in which the compositions disclosed in SEQ ID NOS:1-14 or portions thereof, are-used as the target in a combinatorial or screening protocol.

It is understood that when using the disclosed compositions in combinatorial techniques or screening methods, molecules, such as macromolecular molecules, will be identified that have particular desired properties such as stimulation or the target molecule's function. The molecules identified and isolated when using the disclosed compositions such as STS polypeptides and nucleic acids are also disclosed. Thus, the products produced using the combinatorial or screening approaches that involve the disclosed compositions, such as STS polypeptides and nucleic acids are also disclosed.

### Kits

Disclosed herein are kits that are drawn to reagents that can be used in practicing the methods disclosed herein. The kits can include any reagent or combination of reagent discussed herein or that would be understood to be required or beneficial in the practice of the disclosed methods. For example, the kits could include STS nucleic acids or polypeptides, such as those disclosed herein.

### Compositions with similar functions

It is understood that the compositions disclosed herein have certain functions, such as editing ApoB100. Disclosed herein are certain structural requirements for performing the disclosed functions, and it is understood that there are a variety of structures which can perform the same function which are related to the disclosed structures, and that these structures will ultimately achieve the same result, for example reducing atherogenic risk.

### Methods of making the compositions

The compositions disclosed herein and the compositions necessary to perform the disclosed methods can be made using any method known to those of skill in the art for that particular reagent or compound unless otherwise specifically noted.

### Nucleic acid synthesis

For example, the nucleic acids, such as the STS nucleic acids, can be made using standard chemical synthesis methods or can be produced using enzymatic methods or any other known method. Such methods can range from standard enzymatic digestion followed by nucleotide fragment isolation (see for example, Sambrook et al., Molecular Cloning: A Laboratory Manual, 2nd Edition (Cold Spring Harbor Laboratory Press, Cold Spring Harbor, N.Y., 1989) Chapters 5, 6) to purely synthetic methods, for example, by the cyanoethyl phosphoramidite method using a Milligen or Beckman System 1Plus DNA synthesizer (for example, Model 8700 automated synthesizer of Milligen-Biosearch, Burlington, MA or ABI Model 380B). Synthetic methods useful for making oligonucleotides are also described by Ikuta et al., Ann. Rev. Biochem. 53:323-356 (1984), (phosphotriester and phosphite-triester methods), and Narang et al., Methods Enzymol., 65:610-620 (1980), (phosphotriester method). Protein nucleic acid molecules can be made using known methods such as those described by Nielsen et al., Bioconjug. Chem. 5:3-7 (1994).

### Peptide synthesis

One method of producing the disclosed proteins, such as SEQ ID NO:1, is to link two or more peptides or polypeptides together by protein chemistry techniques. For example, peptides or polypeptides can be chemically synthesized using currently available laboratory equipment using either Fmoc (9-fluorenylmethyloxycarbonyl) or Boc (tert -butyloxycarbonoyl) chemistry. (Applied Biosystems, Inc., Foster City, CA). One skilled in the art can readily appreciate that a peptide or polypeptide corresponding to the disclosed proteins, for example, can be synthesized by standard chemical reactions. For example, a peptide or polypeptide can be synthesized and not cleaved from its synthesis resin whereas the other fragment of a peptide or protein can be synthesized and subsequently cleaved from the resin, thereby exposing a terminal group which is functionally blocked on the other fragment. By peptide condensation reactions, these two fragments can be covalently joined via a peptide bond at their carboxyl and amino termini, respectively, to form an antibody, or fragment thereof. (Grant GA (1992) Synthetic Peptides: A User Guide. W.H. Freeman and Co., N.Y. (1992); Bodansky M and Trost B., Ed. (1993) Principles of Peptide Synthesis. Springer-Verlag Inc., NY . Alternatively, the peptide or polypeptide is independently synthesized in vivo as described herein. Once isolated, these independent peptides or polypeptides may be linked to form a peptide or fragment thereof via similar peptide condensation reactions.

For example, enzymatic ligation of cloned or synthetic peptide segments allow relatively short peptide fragments to be joined to produce larger peptide fragments, polypeptides or whole protein domains (Abrahmsen L et al., Biochemistry, 30:4151 (1991)). Alternatively, native chemical ligation of synthetic peptides can be utilized to synthetically construct large peptides or polypeptides from shorter peptide fragments. This method consists of a two step chemical reaction (Dawson et al. Synthesis of Proteins by Native Chemical Ligation. Science, 266:776-779 (1994)). The first step is the chemoselective reaction of an unprotected synthetic peptide--thioester with another unprotected peptide segment containing an amino-terminal Cys residue to give a thioester-linked intermediate as the initial covalent product. Without a change in the reaction conditions, this intermediate undergoes spontaneous, rapid intramolecular reaction to form a native peptide bond at the ligation site (Baggiolini M et al. (1992) FEBS Lett. 307:97-101; Clark-Lewis I et al., J.Biol.Chem., 269:16075 (1994); Clark-Lewis I et al., Biochemistry, 30:3128 (1991); Rajarathnam K et al., Biochemistry 33:6623-30 (1994)).

Alternatively, unprotected peptide segments can be chemically linked where the bond formed between the peptide segments as a result of the chemical ligation is an unnatural (non-peptide) bond (Schnolzer, M et al. Science, 256:221 (1992)). This technique has been used to synthesize analogs of protein domains as well as large amounts of relatively pure proteins with full biological activity (deLisle Milton RC et al., Techniques in Protein Chemistry IV. Academic Press, New York, pp. 257-267 (1992)).

### Processes for making the compositions

Disclosed are processes for making the compositions as well as making the intermediates leading to the compositions. For example, disclosed are nucleic acids in SEQ ID NOs: 1-5, 8, 10, 12, and 14. There are a variety of methods that can be used for making these compositions, such as synthetic chemical methods and standard molecular biology methods. It is understood that the methods of making these and the other disclosed compositions are specifically disclosed.

Disclosed are nucleic acid molecules produced by the process comprising linking in an operative way a nucleic acid comprising the sequence set forth in SEQ ID NOs:1-5, 8, 10, 12, and 14 and a sequence controlling the expression of the nucleic acid.

Also disclosed are nucleic acid molecules produced by the process comprising linking in an operative way a nucleic acid molecule comprising a sequence having 80% identity to a sequence set forth herein and a sequence controlling the expression of the nucleic acid.

Disclosed are nucleic acid molecules produced by the process comprising linking in an operative way a nucleic acid molecule comprising a sequence that hybridizes under stringent hybridization conditions to a sequence set forth herein and a sequence controlling the expression of the nucleic acid.

Disclosed are nucleic acid molecules produced by the process comprising linking in an operative way a nucleic acid molecule comprising a sequence encoding a peptide set forth herein and a sequence controlling an expression of the nucleic acid molecule.

Disclosed are nucleic acid molecules produced by the process comprising linking in an operative way a nucleic acid molecule comprising a sequence encoding a peptide having 80% identity to a peptide set forth herein and a sequence controlling an expression of the nucleic acid molecule.

Disclosed are nucleic acids produced by the process comprising linking in an operative way a nucleic acid molecule comprising a sequence encoding a peptide having 80% identity to a peptide set forth in any of SEQ ID NOs:6, 7, 9, 11, or 13, wherein any change from the disclosed sequence are conservative changes and a sequence controlling an expression of the nucleic acid molecule.

Disclosed are cells produced by the process of transforming the cell with any of the disclosed nucleic acids. Disclosed are cells produced by the process of transforming the cell with any of the non-naturally occurring disclosed nucleic acids.

Disclosed are any of the disclosed peptides produced by the process of expressing any of the disclosed nucleic acids. Disclosed are any of the non-naturally occurring disclosed peptides produced by the process of expressing any of the disclosed nucleic acids. Disclosed are any of the disclosed peptides produced by the process of expressing any of the non-naturally disclosed nucleic acids.

Disclosed are animals produced by the process of transfecting a cell within the animal with any of the nucleic acid molecules disclosed herein. Disclosed are animals produced by the process of transfecting a cell within the animal any of the nucleic acid molecules disclosed herein, wherein the animal is a mammal. Also disclosed are animals produced by the process of transfecting a cell within the animal any of the nucleic acid molecules disclosed herein, wherein the mammal is mouse, rat, rabbit, cow, sheep, pig, or primate.

Also disclosed are animals produced by the process of adding to the animal any of the cells disclosed herein.

### METHODS

### Methods of using the compositions

Disclosed herein are methods of expressing APOBEC-1 in a cell, comprising bringing into contact a cell and a vector comprising a nucleic acid, wherein the nucleic acid encodes a polypepetide comprising an APOBEC-1 sequence, a secretion sequence and a transduction sequence; whereby the nucleic acid produces APOBEC-1, thereby expressing APOBEC-1 in the cell. The APOBEC-1 polypeptide can then be secreted from the cell and transduce a second cell, thereby delivering the polypeptide to the second cell. This can occur *in vivo* or *in vitro,* such as in culture. The method can take place in a subject, wherein the cell and the vector are brought into contact by administering the vector to the subject, wherein expression of the polypeptide reduces atherogenic risk in the subject.

In one example, the subject can have high levels of serum LDL. Expression of the polypeptide in a subject can also inhibit the binding of low density lipoprotein to blood vessel matrix. Expression of the polypeptide in a subject can also decrease apolipoprotein B-containing lipoprotein or triglyceride, or decrease apoB100. The decrease in apolipoprotein B-containing lipoprotein can require low density lipoprotein receptor activity, for example. High levels of serum LDL include any levels of serum LDL that are higher than normal or healthy levels. For example, levels high levels of serum LDL can be any level of serum LDL that would indicate that the subject be treated for or take action to reduce the srum LDL level. Expression of the polypeptide can also decrease serum LDL. The subject can also be suffering from dyslipidemia or cardiovascular disease, whereby the subject's dyslipidemia or cardiovascular disease is treated by the methods disclosed herein. The dyslipidemia can occur in familial hyperlipidemia, obesity, or diabetes, for example, and the cardiovascular disease can be atherosclerosis, for example.

Secretory signals linked to TAT-HA-APOBEC and gene delivery via a viral vector can be used to generate somatic mosaic expression of TAT-HA-APOBEC for extracellular distribution and uptake by adjacent hepatocytes in the liver. As discussed above, this is useful because, for example: 1) targeting TAT-HA-APOBEC for secretion can prevent the build up of TAT-HA-APOBEC in the producer cell, thereby averting effects to due to overexpression and promiscuous editing; 2) secreted TAT-HA-APOBEC can be rapidly diluted in the intrahepatic but extracellular fluids in the Space of Disse, thereby reducing the probability of transduction of the producer cell; 3) hepatocytes can be transduced by TAT-HA-APOBEC and secrete apoB48-containing VLDL in a dose-dependent manner defined by the concentration gradients established around the producer hepatocytes; and 4) the effect of TAT-HA-APOBEC transduction and induction of apoB mRNA editing activity is localized primarily to the liver due to the anatomy of the intrahepatic circulation, the absence of blood cells in the Space of Disse, the absence of heparin polysaccharides on Kupffer cells and the limited expression of apoB mRNA in other tissues.

### Methods of gene modification and gene disruption

The disclosed compositions and methods can be used for targeted gene disruption and modification in any animal that can undergo these events. Gene modification and gene disruption refer to the methods, techniques, and compositions that surround the selective removal or alteration of a gene or stretch of chromosome in an animal, such as a mammal, in a way that propagates the modification through the germ line of the mammal. In general, a cell can be transformed with a vector which is designed to homologously recombine with a region of a particular chromosome contained within the cell, as for example, described herein. This homologous recombination event can produce a chromosome which has exogenous DNA introduced, for example in frame, with the surrounding DNA. This type of protocol allows for very specific mutations, such as point mutations, to be introduced into the genome contained within the cell. Methods for performing this type of homologous recombination are disclosed herein. Homologous recombination in mammalian cells may require the cells to be cultured, because the desired recombination event occurs at a low frequency.

Once the cell is produced through the methods described herein, an animal can be produced from this cell through either stem cell technology or cloning technology. For example, if the cell into which the nucleic acid was transfected was a stem cell for the organism, then this cell, after transfection and culturing, can be used to produce an organism which will contain the gene modification or disruption in germ line cells, which can then in turn be used to produce another animal that possesses the gene modification or disruption in all of its cells. In other methods for production of an animal containing the gene modification or disruption in all of its cells, cloning technologies can be used. These technologies generally take the nucleus of the transfected cell and either through fusion or replacement fuse the transfected nucleus with an oocyte which can then be manipulated to produce an animal. The advantage of procedures that use cloning instead of ES technology is that cells other-than ES cells can be transfected. For example, a fibroblast cell, which is very easy to culture can be used as the cell which is transfected and has a gene modification or disruption event take place, and then cells derived from this cell can be used to clone a whole animal.

### EXAMPLES

The following examples are put forth so as to provide those of ordinary skill in the art with a complete disclosure and description of how the compounds, compositions, articles, devices and/or methods claimed herein are made and evaluated, and are intended to be purely exemplary and are not intended to limit the disclosure. Efforts have been made to ensure accuracy with respect to numbers (e.g., amounts, temperature, etc.), but some errors and deviations should be accounted for. Unless indicated otherwise, parts are parts by weight, temperature is in °C or is at ambient temperature, and pressure is at or near atmospheric

### Example 1: Reduction of Atherogenic Risk Due to High Levels of Serum LDL.

TAT-APOBEC can be adapted for secretion with a signal peptide (SP) and delivered via an adeno-associated virus vector (rAAV). In this approach, a somatic mosaic for TAT-APOBEC expression is created in the liver by limited AAV-SP-TAT-APOBEC infection. Secretion of TAT-APOBEC by infected hepatocytes limits its accumulation in the producing cells, eliminating the risk of carcinogenesis from excess editing, and transduces RNA-editing activity into nearby uninfected cells thus enhancing the reduction in LDL.

TAT-APOBEC has been engineered with a protein secretion signal from albumin and propose a limited gene therapy strategy using an adeno-associated virus vector (AAV2). Infected cells synthesize and secrete TAT-APOBEC-1 thereby eliminating the potentially carcinogenic off-target effects in the producer cells. Once secreted into the Space ofDisse, TAT-APOBEC-1 diffuses and transduces into neighboring hepatocytes, generating a zone of cells in which a proportion of apoB mRNA becomes edited leading to the intended decrease in apoB100 secretion and the complementary increase in the synthesis of apoB48 VLDL. A therapeutic benefit in LDL reduction is achieved through a low level of infection of the liver leading to a hepatic mosaic for the production of apoB48. Further, because TAT-APOBEC has a different mode of action it can be synergistic with other current therapies for LDL reduction thus enabling a reduction in the therapeutic threshold and a reduction in cost and side-effects.

**Induction of secretion of TAT-APOBEC in human hepatoma cells by infection with AAV- TAT-APOBEC with associated protein secretion signals. APOBEC is normally an** intracellular protein found in the cytoplasm and the nucleus. The amino terminal signal peptide (SP) sequence from albumin are added to the TAT-HA-APOBEC chimera in the context of AAV2. HepG2 cells infected with this construct are evaluated for expression and secretion by western blotting for the HA-tag embedded in TAT-APOBEC.

**Demonstration of the therapeutic efficacy *in vivo.*** Animals can be used to determine how infection dose correlates with efficiency of secretion and uptake using the endpoints of RNA editing and modulation of apoB48 expression *in vivo.* Hamsters are an ideal small mammalian model because, like humans, they do not edit *apoB* mRNA in their livers, and therefore their serum VLDL particles only contain apoB100. Groups of infected animals are examined for optimal infection by AAV2, the presence and distribution of TAT-HA-APOBEC in their livers, hepatic edited apoB mRNA, circulating levels of apoB100 and apoB48, changes in the ratio of LDL-cholesterol to total cholesterol, and hepatic histology.

**Demonstration of the uptake of TAT-HA-APOBEC and transfer of RNA-editing capability by co-culture with uninfected cells.** *In vitro* experiments determining the effect of expression of TAT-HA-APOBEC are performed in parallel with the animal studies. Transduction of *E. coli*-produced TAT-HA-APOBEC is already known. It can be shown that the amount of TAT-HA-APOBEC synthesized and secreted following infection is sufficient to transduce neighboring cells with minimal effects on the producer cell. Assessment of this feature can be addressed by co-culturing a few infected HepG2 human hepatoma cells among a vast excess of uninfected, non-editing McArdle rat hepatoma cells. The proportion and distribution of cells that have TAT-HA-APOBEC in their cytoplasm and nuclei (the site of apoB mRNA editing) can be determined by HA-tag immunohistochemistry. RNA-editing in producer human cells and the transduction of editing activity in the target rat cells can be measured using species-specific primers for apoB mRNA by RT-PCR and poisoned primer extension sequencing to quantify cytidine transition to uridine at nucleotide 6666. VLDL secreted into the culture media can be assayed by western blotting with N-terminal specific ApoB antibodies to determine the ratio of ApoB100 and ApoB48.

**Secretion of TAT-HA-APOBEC.** The features of this example are the secretion of TAT-HA-APOBEC following an AAV-SP-TAT-HA-APOBEC infection, uptake of the secreted TAT-HA-APOBEC by neighboring non-infected hepatocytes, function of the transduced APOBEC-1 measured as induced apoB48 expression *in vitro* and *in vivo*, increased serum apoB48 VLDL and decreased serum apoB100 LDL and liver histology.

*Induction of TAT-APOBEC secretion in human hepatoma cells by infection with AAV-SP-TAT-HA-APOBEC*. TAT-HA-APOBEC is incorporated with the secretion signals from human serum albumin into pcDNA3 and transfecting the insert-containing plasmid into HepG2 cells *in vitro*. This allows more facile iteration of secretion signals should that be required.

*Construction of pcDNA3 recombinant.* APOBEC-1 (NM_001644) is 27 kDa protein encoded by an 891 nucleotide (nt) mRNA. It is an intracellular enzyme that selectively edits apoB mRNA in the nucleus (Yang et al. (2002). The structural elements for detection of the protein and transduction (the HA epitope) have been added to human APOBEC cDNA in the construction of TAT-HA-APOBEC. The 11 amino acid long TAT motif was selected because it has been proven to mediate transmembrane delivery of numerous proteins including APOBEC into hepatoma cells (Yang et al. (2002, Wadia et al. (2005) Adv Drug Deliv Rev 57, 579-596, Brooks et al. (2005) Adv Drug Deliv Rev 57, 559-577).

PCR cloning strategies can be devised for inserting critical elements for targeting of TAT-HA-APOBEC to the ER and secretion. These include the 5'-UTR and signal peptide (SP) sequence (nt 1-111) and 3'-UTR (nt 1867-2155) from human serum albumin (HSA) (NM_000477) flanking the 5' and 3' ends (respectively) of TAT-HA-APOBEC cDNA in pcDNA3. The choice of the HSA signal peptide and 3'-UTR is based on its proven efficacy to drive protein secretion in bacteria and CHO cells (Saunders et al. (1987) J Bacteriol 169, 2917-2925) and HSA is natively made in abundance by the liver. Although the polyadenylation signal available in pcDNA3 is maintained, the 3'UTR of serum albumin is included as this has been shown to enhance targeting for secretion (http://www.actip.org/manuals/filesantwerp/Beate%20Stern.pdf) and contains poly-adenylation signals native to albumin.

*Construction of rAAV2 recombinant.* The cDNA inserted in pcDNA3 is subcloned into pSub201 transfer vector and used to produce rAAV2 via the helper virus free, three-plasmid transfection system as previously described (Ito et al. (2005)). Scale up viral preparation and viral titering is performed commercially by Applied Viromics (www.appliedviromics.com).

*Analysis of TAT-HA-APOBEC secretion in transfected HepG2 cells.* HepG2 cells grown in 6-well cluster dish are transfected with pcDNA3-HSA 5'-UTR-TAT-HA-APOBEC-HSA 3'-UTR construct using Fugene6 and media and 24 hours later the cells can be washed with PBS and mixed at varying dilutions with non transfected HepG2 cells (1/100 to 1/10,000) and replated at their original density for an additional 6-48 hours. At appropriate times, cells can be collected for western blotting analysis using monoclonal mouse anti-HA. Cells can be lysed with Reporter Lysis Buffer (Promega) and 40 µg of cleared extracts can be resolved by 10% SDS-PAGE and blotted onto nitrocellulose. Blots can be reacted with anti-HA, and then developed with peroxidase-conjugated goat anti-mouse antibody and ECL (Amersham). The blots can be stripped and reacted with anti-actin antibodies and developed as described above to control for the amount of total cellular material loaded. Autoradiographs can be quantified by scanning densitometry and the TAT-HA-APOBEC signals in each lane can be normalized relative to the actin. An aliquot of tissue culture media collected at each time point can be acetone-precipitated and analyzed by anti-HA immunoblotting as described above. The TAT-HA-APOBEC signals can be normalized based on the densitometric signals resulting from reprobing the blots with anti-albumin monoclonal antibodies.

The concept in this experiment is that a conventional transfection construct results in recombinant protein expression in a limited number of cells and induces apoB mRNA editing to a limited extent within 48 h. Transfection with a transduction construct followed by dilution of these cells in non transfected cells only results in significant increases in apoB mRNA editing if TAT-HA-APOBEC secretion and transduction has occurred. HepG2 cells routinely have 30-40% transfection efficiencies with pcDNA3-containing APOBEC and without secretory signals APOBEC enters the nucleus and induces apoB mRNA editing to 40% (HepG2 have no editing activity without APOBEC transfection).

### Demonstration of the therapeutic efficacy in vivo.

*Design of the animal infection and functional endpoint studies.* The Syrian golden hamster has been chosen as the model animal because it does not edit apoB mRNA in its liver and has serum a lipoprotein profile similar to humans when fed normal chow (Ranhotra et al. (1996), Davis, P. et al. (2006) J Nutr 136, 428-432). Animals are housed in controlled environments with 12 h light cycle and fed normal hamster chow (Purina). Five groups, each comprised of 24, seven week-old female hamsters, are housed 3 per cage and infected intravenously on Day 1 with: (1) Sham-infected (saline only); (2) rAAV2 with a TAT-β-Galactosidase insert prepared by Applied Viromics, using 5 x 10¹⁰ gene copies per animal; (3) AAV-HSA 5'-UTR-TAT-HA-APOBEC-HSA 3'-UTR, low dose; 5 x 10⁹ gene copies per animal; (4) AAV-HSA 5'-UTR-TAT-HA-APOBEC-HSA 3'-UTR, medium dose; 5 x 10¹⁰ gene copies per animal; and (5) AAV-HSA 5'UTR-TAT-HA-APOBEC-HAS 3' UTR, high dose; 5 x 10¹¹ gene copies per animal. Dose ranges were taken from recommendations by Appied Viromics and adjusted animal body weight (mouse vs hamster). The units, gene copies (GC) instead of the more conventional plaques-forming units (pfu) or multiplicity of infection (moi), are Applied Viromics preferred units.

Three animals from each group are sacrificed at the 1h, 2h, 6h, 24h, 7d, 14d, 30d and 180d time points. These time points are selected so that the immediate effect of infection can be observed as well as the onset of therapeutic benefit and then a more long-term host response evaluated. Animals can be anesthetized using 20 mg sodium pentobarbital per kg BW and maximum blood withdrawn by cardiac puncture using a 18 gauge needle. Most of the liver can be removed and quick frozen in liquid nitrogen for biochemical and histological analysis. One fourth of the liver can be diced and fixed in 4% formalin for immunohistochemical analysis and β-galactosidase histochemical staining. Animals can be sacrificed by cutting the inferior vena cava and cervical dislocation.

*Lipid and lipoprotein analysis.* For each time point, plasma is assayed for: 1) total serum cholesterol and triglyceride, 2) VLDL +LDL ratio to HDL, 3) levels of apoB48 and apoB100 in each lipoprotein fraction and 4) albumin, and TAT-HA-APOBEC in plasma.

ApoB containing lipoproteins can be isolated and purified by standard methods in the field (Nakamuta et al (1998) Arterioscler Thromb Vasc Biol 18, 747-755). Briefly, heparinized blood can be collected and cleared of red and white blood cells. ApoB-containing lipoproteins in the plasma can be isolated by density gradient ultracentrifugation (Beckman 50Ti rotor, 40 000 rpm, 10°C, 8 hours) after adjusting plasma samples to a density of 1.063 g/mL with a KBr solution (d = 1.35 g/mL) in tubes containing a KBr overlay solution (d = 1.063 g/mL). The upper lipoprotein fractions can be dialyzed against 150 mmol/L NaCl, 1 mmol/L EDTA (pH 7.4), and 0.05% NaN3 (wt/vol) and assayed enzymatically for cholesterol using a commercially available kit from Sigma Diagnostics.

Hamster ApoB48 and apoB100 can be quantified by scanning densitometry of Coomassie blue-stained SDS-PAGE as described by Nakamuta et al (1998). Lipoproteins prepared as described above can be resolved on a 2% to 20% linear polyacrylamide gel. The coelectrophoresed immunopurified rat apoB100 and apoB48 serve as a quantification control (Yang et al. (2002)).

Four different density lipoprotein fractions (d < 1.006, d = 1.006 to 1.019, d = 1.019 to 1.063, and d = 1.063 to 1.21 g/mL) can be isolated by ultracentrifugation at 40,000 rpm in a Beckman 50-Ti rotor at 10°C for 20, 24, 36, and 48 hours, respectively. An aliquot from each fraction is assayed for total cholesterol and triglycerides using a commercially available kit from Sigma Diagnostics. The apoB48 and apoB100 profiles of the lipoprotein fractions can be determined by SDS-PAGE and Coomassie blue staining as described above. Protein in each fraction can be quantified using the Bio-Rad protein assay. Relative particle sizes of VLDL (d < 1.006 g/mL), LDL (d = 1.006 to 1.063 g/mL), and HDL (d = 1.063 to 1.21 g/mL) can be determined by non-denaturing gradient gel electrophoresis (Nichols et al.(1986) Methods Enzymol 128, 417-431).

Plasma levels of Albumin and TAT-HA-APOBEC can be determined by immunoblotting using rabbit polyclonal, cross-reacting anti-albumin antibody and anti-HA, respectively.

*ApoB mRNA editing quantification.* ApoB mRNA editing can be determined following reverse transcript PCR (RT-PCR) using the established poisoned-primer extension assay (Smith et al. (1991) Proc Natl Acad Sci USA 88, 1489-1493). Total tissue RNA can be isolated from snap frozen liver samples in TriReagent. cDNA can be synthesized using AMV reverse transcriptase and oligo dT. ApoB-specific primers can be designed that flank the hamster editing site within exon 28 such that a 400 nt PCR product can be amplified. A 5'-end radiolabeled primer complementary to the apoB PCR product (and annealing five nucleotides 3' of the edited nucleotide at nt position 6666) can be subjected to RT primer extension in the presence of dATP, dCTP, TTP and a 1:5 ratio of dGTP to ddGTP. A stop on unedited (C6666) PCR products generates a shorter extension product whereas edited PCR products (U6666) extend to the next in frame C and hence are longer. Percent editing is quantified from Phosphorimage scanning densitometric analysis primer extension products resolved on denaturing gels and is calculated as the amount of long product divided by the amount of long plus short product x 100. The assay has a detection limit of 0.1% editing and a precision of +/- 3-5%.

*Tissue histology.* Sections on glass slides can be fixed with 4% formalin and rinsed in PBS. To visualize the expression and transduction of the control gene β-Galactosidase, sections can be reacted with Xgal and ferro- and ferric-cyanide for color development overnight at room temperature using the in situ β-Galactosidase staining kit from Invitrogen. In liver sections from animals infected with TAT-HA-APOBEC, fixed sections can be blocked with 1% BSA, reacted with anti-HA antibody and, following washes overnight in PBS, reacted with peroxidase-conjugated secondary antibody. Following washes in PBS, the sections can be reacted with chromagen and hydrogen peroxide. All stained sections can be evaluated by low and high power microscopy for: 1) the general levels of β-Galactosidase or TAT-HA-APOBEC; 2) the number of zones within each section containing recombinant protein and the relative dimensions of each zone; and 3) at high power, the distribution of recombinant protein within cells of each zone containing positively staining cells and whether there are differences within the zone suggesting producers cells with only endomembrane positive staining and transduced cells with general cytoplasmic and nuclear staining. The Pathology cytology labs can provide serial sections of livers on slides that enable the assessment of zone dimensions and the intracellular distribution of recombinant proteins within cells.

*Statistical Analysis.* Values can be determined as means and standard deviations. The data can be analyzed using one-way ANOVA and Duncan's new multiple range test using SigmaStat statistical package. Differences are considered significant at P < 0.05.

*TAT-HA-APOBEC distribution.* rAAV can infect 1-10% of hepatocytes following IV injection and within 48 hours, infected hepatocytes begin to secrete TAT-HA-APOBEC. The secreted protein can reside for a short time in the Space of Disse, the plasma-exposed, extracellular matrix-rich gap between the hepatocyes and the fenestrated endothelium characteristic of the liver. The Space of Disse (Figure 1) is a privileged environment for uptake of TAT-HA-APOBEC because it is inaccessible to blood cells, and it is free from the turbulence and the high flow rate of the circulation. Further, it can have a big enough volume to prevent uptake primarily by the producing cells while still limiting dilution so that uptake by nearby uninfected hepatocytes is probable. By staining thin sections of the livers, the distribution of TAT-HA-APOBEC can appear like long thin islands of stained cells running along the hepatic cords and surrounding the local producer cell in a matrix of non-staining cells. Depending on the level of infecting rAAV2, these islands can be discrete like raisins in a pudding or nearly a continuum.

*ApoB mRNA editing.* Cells that stain for TAT-HA-APOBEC can also edit apoB mRNA, a point that can have already been demonstrated in vitro. Samples for measurement of RNA-editing can consist of some TAT-HA-APOBEC containing cells and others that have none so the RNA-editing activity can be averaged over an undefined population. Because the hamster does not edit apoB mRNA, detection of apoB mRNA editing is unhindered by endogenous background.

*Serum lipoproteins.* Transduction of TAT-HA-APOBEC can result in apoB mRNA editing, the translation and assembly of apoB48 VLDL and a rapid increase in apoB48 VLDL with a corresponding reduction in apoB100 VLDL and LDL.

### Demonstration of the uptake of TAT-HA-APOBEC and transfer of RNA-editing capability by co-culture with uninfected cells.

Animal studies demonstrate that the amount of TAT-HA-APOBEC-1 synthesized and secreted following infection is sufficient to transduce neighboring cells and what the effect of expressing HSA 5`UTR-TAT-HA-APOBEC-HSA 3' UTR is on the producer cells either due to reinternalization of TAT-HA-APOBEC following secretion (self transduction) or due to escape of TAT-HA-APOBEC from the endomembrane system and entry into the cytoplasm and nucleus.

*Co-culture experiments.* For these analyses, advantage is taken of the fact that: 1) human HepG2 hepatoma cells and rat McArdle hepatoma cells have very similar media requirements; 2) HepG2 cells produce but do not edit apoB unless transduced by TAT-HA-APOBEC whereas McArdle cells support 14% apoB mRNA editing but respond more robustly when transduced with TAT-HA-APOBEC and can edit apoB mRNA with up to 70% efficiency; and 3) human apoB and rat apoB mRNA within the vicinity of the editing site can be selectively amplified with species-specific primers. The experiment can be conducted in a 6-well culture dish format contain 1 mL of media per well and involve mixing infected with uninfected cells and determine the level of transduction in the culture.

HepG2 cell cultures can be infected at different GC levels with the recombinant virus and after 6-12 hours, the cells can be harvested and washed repeatedly with PBS. Uninfected McArdle cells can be harvested at the same time, mixed with the infected HepG2 cells at a range of high dilutions (1/1,000 to 1/50,000; HepG2/McArdle) and replated. At 24 and 48 hours all the various co-cultures can be extracted in TriReagent and the RNA processed for species-specific apoB RT-PCR and editing quantified by poisoned primer extension as described above. The proportion of cells that have TAT-HA-APOBEC in their cytoplasm and nuclei (the site of apoB mRNA editing) can be determined microscopically following anti-HA immunohistochemistry using FITC-conjugated secondary antibodies. RNA-editing in the McArdle non-infected recipient cells can be measured using species-specific primers for RT-PCR and poisoned primer extension sequencing to quantify cytidine transition to uridine at nucleotide 6666. The amount of editing in McArdle producer cells and the fidelity of TAT-APOBEC-dependent editing of apoB mRNA can be determined by sequence analysis of PCR products. VLDL secreted into the culture media can be assayed by western blotting with rat-specific, N-terminal-reactive apoB monoclonal antibodies to evaluate changes in the ratio of ApoB 100 and ApoB48.

The high dilution of infected HepG2 cells relative to co-cultured McArdle cells is intended to mimic the dynamics of a producer cell secreting TAT-HA-APOBEC into the Space of Disse (a relatively large compartment). Microscopically, HepG2 cells are rounded and 'eye' shaped, and can be distinguished from McArdle cells which are flatten and have 'spread-out' cytoplasms. This, together with co-immunostaining for human-specific surface antigens, can assist in ascribing the TAT-HA-APOBEC intracellular distributions to producer and recipient cells.

### VERTEBRATE ANIMALS

*Hamsters.* Female Syrian golden hamsters seven weeks of age; (Harlan, Indianapolis, IN) fed ad libitum on basal diet purchased from Dyets (Bethlehem, PA). Animals can be housed 3 individuals per cage and maintained under 12-hour regulated lighting and temperature conditions.

*rAAV2 infection.* Each animal receives a single injection of 0.1 cc of rAAV in phosphate-buffered saline (PBS) or PBS alone on day zero of the experiment. rAAV2-TAT-APOBEC and rAAV2-TAT-β-galactosidase stocks can be prepared commercially by Applied Viromics (www.appliedviromics.com) and diluted with PBS to the appropriate concentration. Hamsters can be injected intravenously with an injection volume of 0.1 cc in PBS. Infection with rAAV2 constructs can be according to Naki et al., (J. Virol., 1999, 73:5438-47) using 5 x 10⁹, 5 x 10¹⁰ or 5 x 10¹¹ gene copies (GC)/injection/animal. Alternatively, animals can be injected with the described doses in the portal vein. After placing the animals under sodium pentobarbital anesthesia (40 mg sodium pentobarbital per kg BW), a ventral incision can be made to expose the hepatic portal vein and PBS or virus can be delivered via a 30 gauge needle. Naki *et al* realized a 5-10 fold higher hepatic infection through this alternate route.

*Experimental procedure and termination.* Following rAAV2 infection, animals can be fasted and liberated of pouch-stored food for 6 h before blood is collected under anesthesia by cardiac puncture in a tube containing EDTA and the animal sacrificed by cervical dislocation and surgical severing of the dorsal aorta. Sacrificed animals can be dissected and liver, small intestine, kidney, muscle and brain quick frozen for sectioning and histological or biochemical evaluation. Serum lipoproteins and tissue can be evaluated at 1h, 2h, 6h, 24h, 48h, 7d, 14d, 30d and 180d time points. There are two control groups (PBS injected only and rAAV containing beta-galactosidase). With two controls, three animals per group, and nine time points, 54 total animals are used for controls. For the experimental animals, three different dose levels are tested, with three animals per level, at each of nine time points, so 81 total animals are used as experimentals.

*Statistics.* Values are determined as means and standard deviations. The data is analyzed using one-way ANOVA and Duncan's new multiple range test using SigmaStat statistical package. Differences are considered significant at *P* < 0.05.

### References

1. Corsetti, J.P. et al. Metabolic syndrome best defines the multivariate distribution of blood variables in postinfarction patients. Atherosclerosis 171, 351-358 (2003).
2. Gibbons, G.H. et al. Genetic markers: progress and potential for cardiovascular disease. Circulation 109, IV47-58 (2004).
3. Scott, J. The molecular and cell biology of apolipoprotein-B. Mol Biol Med 6, 65-80 (1989).
4. Powell, L.M. et al. A novel form of tissue-specific RNA processing produces apolipoprotein-B48 in intestine. Cell 50, 831-840 (1987).
5. Chen, S.H. et al. Apolipoprotein B-48 is the product of a messenger RNA with an organ-specific in-frame stop codon. Science 238, 363-366 (1987).
6. Johnson, D.F., Poksay, K.S. & Innerarity, T.L. The mechanism for apo-B mRNA editing is deamination. Biochem Biophys Res Commun 195, 1204-1210 (1993).
7. Greeve, J., Altkemper, I., Dieterich, J.H., Greten, H. & Windler, E. Apolipoprotein B mRNA editing in 12 different mammalian species: hepatic expression is reflected in low concentrations of apoB-containing plasma lipoproteins. J Lipid Res 34, 1367-1383 (1993).
8. Backus, J.W. et al. Quantitation of endogenous liver apolipoprotein B mRNA editing. Biochem Biophys Res Commun 170, 513-518 (1990).
9. Sparks, C.E., Hnatiuk, O. & Marsh, J.B. Hepatic and intestinal contribution of two forms of apolipoprotein B to plasma lipoprotein fractions in the rat. Can J Biochem 59, 693-699 (1981).
10. Hirano, K. et al. Targeted disruption of the mouse apobec-1 gene abolishes apolipoprotein B mRNA editing and eliminates apolipoprotein B48. J Biol Chem 271, 9887-9890 (1996).
11. Nakamuta, M. et al. Complete phenotypic characterization of apobec-1 knockout mice with a wild-type genetic background and a human apolipoprotein B transgenic background, and restoration of apolipoprotein B mRNA editing by somatic gene transfer of Apobec-1. J Biol Chem 271, 25981-25988 (1996).
12. Xie, Y., Nassir, F., Luo, J., Buhman, K. & Davidson, N.O. Intestinal lipoprotein assembly in apobec-1-/- mice reveals subtle alterations in triglyceride secretion coupled with a shift to larger lipoproteins. Am J Physiol Gastrointest Liver Physiol 285, G735-746 (2003).
13. Qian, X. et al. Low expression of the apolipoprotein B mRNA-editing transgene in mice reduces LDL levels but does not cause liver dysplasia or tumors. Arterioscler Thromb Vasc Biol 18, 1013-1020 (1998).
14. Teng, B. et al. Adenovirus-mediated gene transfer of rat apolipoprotein B mRNA-editing protein in mice virtually eliminates apolipoprotein B-100 and normal low density lipoprotein production. J Biol Chem 269, 29395-29404 (1994).
15. Hughes, S.D., Rouy, D., Navaratnam, N., Scott, J. & Rubin, E.M. Gene transfer of cytidine deaminase apoBEC-1 lowers lipoprotein(a) in transgenic mice and induces apolipoprotein B editing in rabbits. Hum Gene Ther 7, 39-49 (1996).
16. Navaratnam, N., Shah, R., Patel, D., Fay, V. & Scott, J. Apolipoprotein B mRNA editing is associated with UV crosslinking of proteins to the editing site. Proc Natl Acad Sci USA 90, 222-226 (1993).
17. Dance, G.S. et al. Two proteins essential for apolipoprotein B mRNA editing are expressed from a single gene through alternative splicing. J Biol Chem 277, 12703-12709 (2002).
18. Giannoni, F. et al. Complementation of apolipoprotein B mRNA editing by human liver accompanied by secretion of apolipoprotein B48. J Biol Chem 269, 5932-5936 (1994).
19. Sowden, M., Hamm, J.K. & Smith, H.C. Overexpression of APOBEC-1 results in mooring sequence-dependent promiscuous RNA editing. J Biol Chem 271, 3011-3017 (1996).
20. Teng, B., Burant, C.F. & Davidson, N.O. Molecular cloning of an apolipoprotein B messenger RNA editing protein. Science 260, 1816-1819 (1993).
21. Yamanaka, S., Poksay, K.S., Balestra, M.E., Zeng, G.Q. & Innerarity, T.L. Cloning and mutagenesis of the rabbit ApoB mRNA editing protein. A zinc motif is essential for catalytic activity, and noncatalytic auxiliary factor(s) of the editing complex are widely distributed. J Biol Chem 269, 21725-21734 (1994).
22. Nakamuta, M. et al. Alternative mRNA splicing and differential promoter utilization determine tissue-specific expression of the apolipoprotein B mRNA-editing protein (Apobecl) gene in mice. Structure and evolution of Apobec1 and related nucleoside/nucleotide deaminases. J Biol Chem 270, 13042-13056 (1995).
23. Lau, P.P., Zhu, H.J., Baldini, A., Charnsangavej, C. & Chan, L. Dimeric structure of a human apolipoprotein B mRNA editing protein and cloning and chromosomal localization of its gene. Proc Natl Acad Sci USA 91, 8522-8526 (1994).
24. Hadjiagapiou, C., Giannoni, F., Funahashi, T., Skarosi, S.F. & Davidson, N.O. Molecular cloning of a human small intestinal apolipoprotein B mRNA editing protein. Nucleic Acids Res 22, 1874-1879 (1994).
25. Anant, S., Yu, H. & Davidson, N.O. Evolutionary origins of the mammalian apolipoproteinB RNA editing enzyme, apobec-1: structural homology inferred from analysis of a cloned chicken small intestinal cytidine deaminase. Biol Chem 379, 1075-1081 (1998).
26. Mian, I.S., Moser, M.J., Holley, W.R. & Chatterjee, A. Statistical modelling and phylogenetic analysis of a deaminase domain. J Comput Biol 5, 57-72 (1998).
27. Wedekind, J.E., Dance, G.S., Sowden, M.P. & Smith, H.C. Messenger RNA editing in mammals: new members of the APOBEC family seeking roles in the family business. Trends Genet 19, 207-216 (2003).
28. Sowden, M.P., Ballatori, N., Jensen, K.L., Reed, L.H. & Smith, H.C. The editosome for cytidine to uridine mRNA editing has a native complexity of 27S: identification of intracellular domains containing active and inactive editing factors. J Cell Sci 115, 1027-1039 (2002).
29. Mehta, A., Kinter, M.T., Sherman, N.E. & Driscoll, D.M. Molecular cloning of apobec-1 complementation factor, a novel RNA-binding protein involved in the editing of apolipoprotein B mRNA. Mol Cell Biol 20, 1846-1854 (2000).
30. Lellek, H. et al. Purification and molecular cloning of a novel essential component of the apolipoprotein B mRNA editing enzyme-complex. J Biol Chem 275, 19848-19856 (2000).
31. Shah, R.R. et al. Sequence requirements for the editing of apolipoprotein B mRNA. J Biol Chem 266, 16301-16304 (1991).
32. Mehta, A. & Driscoll, D.M. A sequence-specific RNA-binding protein complements apobec-1 To edit apolipoprotein B mRNA. Mol Cell Biol 18, 4426-4432 (1998).
33. Harris, S.G. et al. Extract-specific heterogeneity in high-order complexes containing apolipoprotein B mRNA editing activity and RNA-binding proteins. J Biol Chem 268, 7382-7392 (1993).
34. Driscoll, D.M., Lakhe-Reddy, S., Oleksa, L.M. & Martinez, D. Induction of RNA editing at heterologous sites by sequences in apolipoprotein B mRNA. Mol Cell Biol 13, 7288-7294 (1993).
35. Smith, H.C. Analysis of protein complexes assembled on apolipoprotein B mRNA for mooring sequence-dependent RNA editing. Methods 15, 27-39 (1998).
36. Sowden, M.P., Eagleton, M.J. & Smith, H.C. Apolipoprotein B RNA sequence 3' of the mooring sequence and cellular sources of auxiliary factors determine the location and extent of promiscuous editing. Nucleic Acids Res 26, 1644-1652 (1998).
37. Siddiqui, J.F., Van Mater, D., Sowden, M.P. & Smith, H.C. Disproportionate relationship between APOBEC-1 expression and apolipoprotein B mRNA editing activity. Exp Cell Res 252, 154-164 (1999).
38. Yamanaka, S. et al. Apolipoprotein B mRNA-editing protein induces hepatocellular carcinoma and dysplasia in transgenic animals. Proc Natl Acad Sci USA 92, 8483-8487 (1995).
39. Yamanaka, S., Poksay, K.S., Arnold, K.S. & Innerarity, T.L. A novel translational repressor mRNA is edited extensively in livers containing tumors caused by the transgene expression of the apoB mRNA-editing enzyme. Genes Dev 11, 321-333 (1997).
40. Strauss, E. Introducing proteins into the body's cells. Science 285, 1466-1467 (1999).
41. Schwarze, S.R., Ho, A., Vocero-Akbani, A. & Dowdy, S.F. In vivo protein transduction: delivery of a biologically active protein into the mouse. Science 285, 1569-1572 (1999).
42. Nagahara, H. et al. Transduction of full-length TAT fusion proteins into mammalian cells: TAT-p27Kip1 induces cell migration. Nat Med 4, 1449-1452 (1998).
43. Yang, Y., Ballatori, N. & Smith, H.C. Apolipoprotein B mRNA editing and the reduction in synthesis and secretion of the atherogenic risk factor, apolipoprotein B100 can be effectively targeted through TAT-mediated protein transduction. Mol Pharmacol 61, 269-276 (2002).
44. Partridge, K.A., Johannessen, A., Tauler, A., Pryme, I.F. & Heskreth, J.E. Competition between the signal sequence and a 3'UTR localisation signal during redirection of beta-globin mRNA to the endoplasmic reticulum: implications for biotechnology. Cytotechnology 30, 37-47 (1999).
45. Ranhotra, G.S., Gelroth, J.A. & Glaser, B.K. Effect of Resistant Starch on Blood and Liver Lipids in Hamsters. Cereal Chemistry 73, 176-178 (1996).
46. Ebrahim, S. et al. What role-for- statins? A review and-economic-model. Health Technol Assess 3, i-iv, 1-91 (1999).
47. Baigent, C. et al. Efficacy and safety of cholesterol-lowering treatment: prospective meta-analysis of data from 90,056 participants in 14 randomised trials of statins. Lancet 366, 1267-1278 (2005).
48. Krasuski, R.A. et al. Conversion to atorvastatin in patients intolerant or refractory to simvastatin therapy: the CAPISH study. Mayo Clin Proc 80, 1163-1168 (2005).
49. Ito, H. et al. Remodeling of cortical bone allografts mediated by adherent rAAV-RANKL and VEGF gene therapy. Nat Med 11, 291-297 (2005).
50. Manno, C.S. et al. Successful transduction of liver in hemophilia by AAV-Factor IX and limitations imposed by the host immune response. Nat Med 12, 342-347 (2006).
51. Vasileva, A. & Jessberger, R. Precise hit: adeno-associated virus in gene targeting. Nat Rev Microbiol 3, 837-847 (2005).
52. Maheshri, N., Koerber, J.T., Kaspar, B.K. & Schaffer, D.V. Directed evolution of adeno-associated virus yields enhanced gene delivery vectors. Nat Biotechnol 24, 198-204 (2006).
53. Fischer, A. & Cavazzanna-Calvo, M. in The Scientist 36-412006).
54. Wadia, J.S. & Dowdy, S.F. Transmembrane delivery of protein and peptide drugs by TAT-mediated transduction in the treatment of cancer. Adv Drug Deliv Rev 57, 579-596 (2005).
55. Brooks, H., Lebleu, B. & Vives, E. Tat peptide-mediated cellular delivery: back to basics. Adv Drug Deliv Rev 57, 559-577 (2005).
56. Saunders, C.W., Schmidt, B.J., Mallonee, R.L. & Guyer, M.S. Secretion of human serum albumin from Bacillus subtilis. J Bacteriol 169, 2917-2925 (1987).
57. Tyagi, M., Rusnati, M., Presta, M. & Giacca, M. Internalization of HIV-1 tat requires cell surface heparan sulfate proteoglycans. J Biol Chem 276, 3254-3261 (2001).
58. Barka, T., Gresik, E.S. & Henderson, S.C. Production of cell lines secreting TAT fusion proteins. J Histochem Cytochem 52, 469-477 (2004).
59. Davis, P. et al. Walnuts reduce aortic ET-1 mRNA levels in hamsters fed a high-fat, atherogenic diet. J Nutr 136, 428-432 (2006).
60. Nakamuta, M., Taniguchi, S., Ishida, B.Y., Kobayashi, K. & Chan, L. Phenotype interaction of apobec-1 and CETP, LDLR, and apoE gene expression in mice: role of apoB mRNA editing in lipoprotein phenotype expression. Arterioscler Thromb Vasc Biol 18, 747-755 (1998).
61. Nichols, A.V., Krauss, R.M. & Musliner, T.A. Nondenaturing polyacrylamide gradient gel electrophoresis. Methods Enzymol 128, 417-431 (1986).
62. Smith, H.C. et al. In vitro apolipoprotein B mRNA editing: identification of a 27S editing complex. Proc Natl Acad Sci USA 88, 1489-1493 (1991).

## Claims

1. Method of expressing APOBEC-1 in a cell in vitro, comprising bringing into contact a cell and a vector comprising a nucleic acid, wherein the nucleic acid encodes a polypeptide comprising an APOBEC-1 sequence, a secretion sequence and a transduction sequence; whereby the nucleic acid produces APOBEC-1, thereby expressing APOBEC-1 in the cell, and wherein the polypeptide is secreted from the cell, and wherein the polypeptide transduces a second cell, thereby delivering the polypeptide to the second cell.

2. A vector comprising a nucleic acid, wherein the nucleic acid encodes a polypeptide comprising an APOBEC-1 sequence, a secretion sequence and a transduction sequence; whereby the nucleic acid produces APOBEC-1, for use for expressing APOBEC-1 in a cell with which the vector is in contact in a subject.

3. The vector of claim 2 for use in a subject having high levels of serum LDL.

4. The vector of claim 2 for use for decreasing apoB100 or for decreasing serum LDL.

5. The vector of claim 2 for use for treating dyslipidemia, particularly familial hyperlipidemia, obesity, or diabetes; or for use for treating cardiovascular disease, particularly atherosclerosis.

6. A polypeptide comprising an APOBEC-1 sequence, a secretion sequence and a transduction sequence.

7. The polypeptide of claim 6, wherein the APOBEC-1 sequence, secretion sequence, and transduction sequence are operably linked; or wherein the APOBEC-1 sequence, secretion sequence, and transduction sequence are contiguous, which polypeptide comprises SEQ ID NO:1, an amino acid sequence at least about 90% identical to the amino acid sequence of SEQ ID NO:1 having apolipoprotein B mRNA editing function, or the amino acid sequence of SEQ ID NO:1 having one or more conservative amino acid substitution having apolipoprotein B mRNA editing function and the signal sequence is an albumin signal sequence: or wherein the signal sequence is selected from the SIGPEP database; and the transduction sequence is TAT.

8. Composition comprising the polypeptide of claim 7.

9. A nucleic acid encoding the polypeptide of claim 7.

10. A vector comprising the nucleic acid of 9.

11. A cell comprising the vector of claim 10.

12. The cell of claim 11, wherein the cell is a hepatocyte.

13. A non-human transgenic animal comprising the cell of claim 11.

14. The vector of claim 10, wherein the nucleic acid encoding the polypeptide is operably linked to an expression control sequence.

## Patentansprüche

1. Verfahren zur Expression von APOBEC-1 in einer Zelle in vitro, welches umfasst, dass eine Zelle und ein Vektor, welcher eine Nukleinsäure aufweist, wobei die Nukleinsäure ein Polypeptid kodiert, welches eine APOBEC-1-Sequenz, eine Sekretionssequenz und eine Transduktionssequenz aufweist, in Kontakt gebracht werden; wobei die Nukleinsäure APOBEC-1 herstellt, wodurch APOBEC-1 in der Zelle exprimiert wird, und wobei das Polypeptid aus der Zelle sekretiert wird, und wobei das Polypeptid eine zweite Zelle transduziert, wodurch das Polypeptide an die zweite Zelle geliefert wird.

2. Vektor, welcher eine Nukleinsäure aufweist, wobei die Nukleinsäure ein Polypeptid kodiert, welches eine APOBEC-1-Sequenz, eine Sekretionssequenz und eine Transduktionssequenz aufweist, wobei die Nukleinsäure APOBEC-1 herstellt, zur Verwendung zur Expression von APOBEC-1 in einer Zelle, mit welcher der Vektor in einem Wesen in Kontakt ist.

3. Vektor nach Anspruch 2 zur Verwendung in einem Wesen, welches hohe Serum-LDL-Spiegel hat.

4. Vektor nach Anspruch 2 zur Verwendung zur Senkung von apoB100 oder zur Senkung von Serum-LDL.

5. Vektor nach Anspruch 2 zur Verwendung zur Behandlung von Fettstoffwechselstörung, insbesondere von familiärer Hyperlipidämie, Fettsucht, oder Diabetes; oder zur Verwendung zur Behandlung von Herz-Kreislauf-Erkrankung, insbesondere Atherosklerose.

6. Polypeptid, welches eine APOBEC-1-Sequenz, eine Sekretionssequenz und eine Transduktionssequenz aufweist.

7. Polypeptid nach Anspruch 6, wobei die APOBEC-1-Sequenz, die Sekretionssequenz und die Transduktionssequenz in operativer Weise verbunden sind; oder wobei die APOBEC-1-Sequenz, die Sekretionssequenz und die Transduktionssequenz zusammenhängend sind; wobei das Polypeptid SEQ ID NO:1, eine Aminosäuresequenz, welche zu mindestens etwa 90% identisch mit der Aminosäuresequenz von SEQ ID NO:1 ist, und welche eine "apolipoprotein B mRNA editing"-Funktion hat, oder die Aminosäuresequenz von SEQ ID NO:1, welche eine oder mehrere konservative Aminosäuresubstitutionen, und welche eine "apolipoprotein B mRNA editing"-Funktion hat; aufweist; und wobei die Signalsequenz eine Albuminsignalsequenz ist; oder wobei die Signalsequenz aus der SIGPEP Datenbank ausgewählt ist; und wobei die Transduktionssequenz TAT ist.

8. Zusammensetzung, welche das Polypeptid nach Anspruch 7 aufweist.

9. Nukleinsäure, welche das Polypeptid nach Anspruch 7 kodiert.

10. Vektor, welcher die Nukleinsäure nach Anspruch 9 aufweist.

11. Zelle, die den Vektor nach Anspruch 10 aufweist.

12. Zelle nach Anspruch 11, wobei die Zelle eine Leberzelle ist.

13. Nicht-menschliches transgenes Tier, welches die Zelle nach Anspruch 11 aufweist.

14. Vektor nach Anspruch 10, wobei die Nukleinsäure, welche das Polypeptid kodiert, in operativer Weise mit einer Expressionskontrollsequenz verbunden ist.

## Revendications

1. Procédé d'expression d'APOBEC-1 dans une cellule in vitro, comprenant la mise en contact d'une cellule et d'un vecteur comprenant un acide nucléique, dans lequel l'acide nucléique code un polypeptide comprenant une séquence d'APOBEC-1, une séquence de sécrétion et une séquence de transduction ; grâce à quoi l'acide nucléique produit l'APOBEC-1, en exprimant de cette manière l'APOBEC-1 dans la cellule, et dans lequel le polypeptide est sécrété à partir de la cellule, et dans lequel le polypeptide transduit une deuxième cellule, en fournissant ainsi le polypeptide à la deuxième cellule.

2. Vecteur comprenant un acide nucléique, dans lequel l'acide nucléique code un polypeptide comprenant une séquence d'APOBEC-1, une séquence de sécrétion et une séquence de transduction ; grâce à quoi l'acide nucléique produit l'APOBEC-1, en vue d'une utilisation pour une expression d'APOBEC-1 dans une cellule avec laquelle le vecteur est en contact chez un sujet.

3. Vecteur selon la revendication 2 destiné à une utilisation chez un sujet présentant des niveaux élevés de lipoprotéine LDL de sérum.

4. Vecteur selon la revendication 2 destiné à une utilisation pour une diminution d'apoB100 ou pour une diminution de lipoprotéine LDL de sérum.

5. Vecteur selon la revendication 2 destiné à une utilisation pour un traitement d'une dyslipidémie, en particulier d'une hyperlipidémie familiale, d'une obésité, ou d'un diabète ; ou destiné à une utilisation pour un traitement d'une maladie cardiovasculaire, en particulier d'une athérosclérose.

6. Polypeptide comprenant une séquence d'APOBEC-1, une séquence de sécrétion et une séquence de transduction.

7. Polypeptide selon la revendication 6, dans lequel la séquence d'APOBEC-1, la séquence de sécrétion, et la séquence de transduction sont liées de manière fonctionnelle ; ou dans lequel la séquence d'APOBEC-1, la séquence de sécrétion, et la séquence de transduction sont contiguës, lequel polypeptide comprend une séquence à numéro d'identification SEQ ID n° :1, une séquence d'acides aminés identique à au moins environ 90 % à la séquence d'acides aminés de la séquence à numéro d'identification SEQ ID n° :1 présentant une jonction de correction à ARNm d'apolipoprotéine B, ou la séquence d'acides aminés de la séquence à numéro d'indentification SEQ ID n° 1 présentant une ou plusieurs substitutions d'acides aminés conservatrices présentant une jonction de correction à ARNm d'apolipoprotéine B et la séquence signal est une séquence signal d'albumine ; ou dans lequel la séquence signal est sélectionnée à partir de la base de données SIGPEP ; et la séquence de transduction est TAT.

8. Composition comprenant le polypeptide selon la revendication 7.

9. Acide nucléique codant le polypeptide selon la revendication 7.

10. Vecteur comprenant l'acide nucléique selon la revendication 9.

11. Cellule comprenant le vecteur selon la revendication 10.

12. Cellule selon la revendication 11, où la cellule est un hépatocyte.

13. Animal transgénique non humain comprenant la cellule selon la revendication 11.

14. Vecteur selon la revendication 10, dans lequel l'acide nucléique codant le polypeptide est lié de manière fonctionnelle à une séquence témoin d'expression.
